(19) Europäisches Patentamt European Patent Office Office européen des brevets

(11) **EP 4 699 652 A1**

(12) **EUROPEAN PATENT APPLICATION**

(43) Date of publication:
25.02.2026 Bulletin 2026/09

(21) Application number: 25174569.1

(22) Date of filing: 06.05.2025

(51) International Patent Classification (IPC):
**A61N 7/00** *(2006.01)*      **A61B 34/00** *(2016.01)*
**A61B 5/00** *(2006.01)*      A61B 34/10 *(2016.01)*

(52) Cooperative Patent Classification (CPC):
**A61N 7/00; A61B 5/4836; A61B 34/25;** A61B 5/05;
A61B 5/369; A61B 5/377; A61B 5/389;
A61B 5/4064; A61B 8/0808; A61B 2034/105;
A61B 2034/107; A61B 2034/252; A61B 2090/374;
A61B 2090/3762; A61B 2090/378;          (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: 21.08.2024 US 202463685512 P
17.01.2025 US 202519029125

(71) Applicant: Sanmai Technologies, PBC
Sunnyvale, CA 94087 (US)

(72) Inventors:
• SATO, TOMOKAZU
SUNNYVALE, CA, 94087 (US)
• DAFT, CHRISTOPHER
SUNNYVALE, CA, 94087 (US)
• SATIR, SARP
SUNNYVALE, CA, 94087 (US)

(74) Representative: Araujo, Daniel
Ingenias Creaciones
Signos e Invenciones S.L.
Avda Diagonal 514 Planta 1, Puerta 4
08006 Barcelona (ES)

(54) **ANATOMICALLY DRIVEN TRANSCRANIAL ULTRASOUND STIMULATION SYSTEM**

(57) The invention relates to a transcranial ultrasound stimulation system (100) and a computer-implemented method for controlling ultrasound stimulation in a subject's brain. The system comprises at least one ultrasound transducer (120) configured to transmit ultrasound signals into brain tissue, a physiological measurement system (140) for monitoring physiological data, and a control unit (130). The control unit (130) is configured to receive a stimulation objective and target brain region, and to determine ultrasound stimulation parameters based on anatomical data and physiological feedback. The control unit (130) further determines a placement and an angular pose for the at least one ultrasound transducer (120), and may generate control signals to induce spatially localized mechanical strain, and optimize field shape or direction according to anatomical orientation, such as axonal or white matter tract alignment. The system may use simulations, test pulses, or evoked responses to refine targeting accuracy. Neuromodulation may be achieved through direct stimulation or by mechanically influencing connected regions.

FIG. 1A

FIG. 1B

(52) Cooperative Patent Classification (CPC): (Cont.)
A61N 2007/0021; A61N 2007/0026;
A61N 2007/0052; A61N 2007/0073;
A61N 2007/0078; A61N 2007/0086

**Description**

TECHNICAL FIELD

The present invention relates to transcranial ultrasound stimulation systems and methods. In particular, the invention concerns ultrasound stimulation systems configured to target brain regions based on anatomical information and to control stimulation parameters dynamically using real-time feedback derived from physiological measurements and imaging data.

PRIOR ART

Transcranial ultrasound (TUS) systems use ultrasound energy to modulate neural activity within the brain through the skull, without surgery or other invasive methods. Current transcranial ultrasound systems typically aim to produce a focus or pressure maximum at the desired treatment location, similar to diagnostic ultrasound imaging systems. In particular, the goal of such systems is often to maintain the tightest possible focus as measured by pressure or intensity.

However, this conventional approach of generating maximum pressure at the target brain structure with the tightest focus has significant limitations. Although it can achieve some clinical effects, it is inefficient because it requires higher ultrasonic intensities than may be necessary for therapeutic efficacy. The observed clinical efficacy may be more a side effect of creating a tight focus than the result of optimal therapeutic stimulation.

For effective neuromodulation, it is important that the ultrasound field can stretch target neuronal structures, rather than simply applying maximum pressure. The stretching of neurons is thought to open ion channels, leading to changes in neural activity. Therefore, ultrasound stimulation parameters should be optimized to maximize stretching rather than pressure. Moreover, the directionality of the ultrasound field relative to anatomical structures is critical: an ultrasound force that merely moves brain tissue without creating tension between anchored structures may be ineffective.

In addition, studies have shown that modifying timing parameters, such as the pulse repetition frequency (PRF), can significantly affect the efficacy and reliability of ultrasound neuromodulation. However, these parameters are often tuned by trial and error, which is time-consuming and can be suboptimal, particularly given the time constraints in clinical and research settings.

There is therefore a need for improved transcranial ultrasound stimulation systems that dynamically adapt stimulation parameters based on real-time physiological feedback, that take into account anatomical features such as fiber/tract orientation and connectivity, and that optimize the ultrasound field characteristics, including field shape, field direction, waveform timing, and other parameters, to maximize mechanical effects such as neuronal stretching, while improving stimulation efficiency and reliability.

SUMMARY OF THE INVENTION

A first aspect of the invention refers to a transcranial ultrasound stimulation system for controlling transcranial ultrasound stimulation. The system does not require a therapeutic outcome and is configured to guide stimulation patterns for neuromodulation or modulation of brain activity without necessarily achieving clinical treatment. The system comprises at least one ultrasound transducer configured to be placed on a subject's head for transmitting ultrasound signals into brain tissue, a physiological measurement system configured to monitor physiological data from the subject, and a control unit. The physiological data may comprise at least one of electroencephalography (EEG), magnetoencephalography (MEG), electromyography (EMG) signals, or imaging data (e.g. captured by the at least one ultrasound transducer).

The control unit comprises at least one processor and an application comprising instructions which, when executed by the at least one processor, cause the system to receive an input indicating a selected stimulation objective and a target brain region for receiving the stimulation. The control unit is further configured to determine ultrasound stimulation parameters for the at least one ultrasound transducer based on anatomical data associated with the selected target brain region and the selected stimulation objective. The stimulation objective may refer to a desired modulation effect in the target brain region, such as excitation, inhibition, or alteration of neuronal activity patterns.

The anatomical data associated with the selected target brain region may comprise imaging data, anatomical models, or structural information describing the morphology or spatial properties of the brain. In some embodiments, the anatomical data may further correspond to predetermined information associating brain regions with suitable stimulation parameters for achieving predetermined stimulation objectives. Such predetermined information may be made available to the application, for example by being stored in the control unit, included as part of the application, or remotely accessible by the control unit. The association between anatomical structures and stimulation parameters may be established through a previous training procedure or based on a database. In certain embodiments, the application may be configured to operate as a trained model for determining ultrasound stimulation parameters based on the anatomical data.

The ultrasound stimulation parameters may include one or more of a focal depth, a pressure to be applied, stimulation timing, or characteristics of the ultrasound waveform (e.g. frequency, amplitude, etc.). The control unit further determines a placement and an angular pose of the at least one ultrasound transducer and outputs the determined placement and angular pose to a user via a user interface.

The control unit is further configured to generate control signals for the at least one ultrasound transducer based on the determined ultrasound stimulation parameters. The control signals cause the at least one ultrasound transducer to transmit an ultrasound waveform. The transmitted ultrasound waveform generates an ultrasound field adapted to apply an ultrasound radiation force within the target brain region. The ultrasound field may be configured not merely to achieve maximum pressure, but rather to induce mechanical effects such as stretching of neuronal structures within the brain tissue, in order to modulate neural activity.

In some embodiments, the transcranial ultrasound stimulation system may comprise a plurality of ultrasound transducers. The determination (e.g. by the control unit) of the ultrasound stimulation parameters for the at least one ultrasound transducer may further comprise selecting a number of ultrasound transducers of the plurality of ultrasound transducers for use (e.g. to be selectively activated) in achieving the selected stimulation objective. The selection process may include selecting one or more sets or subsets of ultrasound transducers to be used for stimulation, for imaging, or for combined stimulation and imaging, and determining corresponding placement locations and angular poses for the selected transducers. By enabling dynamic selection and configuration of multiple transducers, the system may flexibly adapt the ultrasound field characteristics to different anatomical targets and stimulation objectives.

In some embodiments, the control unit may be further configured to generate guidance information for arranging the at least one ultrasound transducer at the determined placement and angular pose based on real-time physiological data monitored by the physiological measurement system. The guidance information may be presented to a user via a user interface and may assist (e.g. by providing information via a display of the user interface) in manually or semi-automatically positioning the at least one ultrasound transducer according to the computed placement and pose. Further, alternatively or complementarily, the control unit may be configured to determine, and/or dynamically update, the placement (e.g. the determination of the placement) and the angular pose of the at least one ultrasound transducer based on real-time feedback from the physiological measurement system and/or from imaging data acquired by the at least one ultrasound transducer. The dynamic updating may enable automatic or user-guided corrections to the transducer position or orientation during the stimulation procedure, thereby improving targeting accuracy based on the subject's real-time physiological state or anatomical imaging feedback.

In some embodiments, the control unit may be further configured to adapt the ultrasound stimulation parameters or the control signals based on real-time feedback derived from physiological data monitored by the physiological measurement system (e.g. by electroencephalography (EEG), magnetoencephalography (MEG), electromyography (EMG) signals, or imaging data captured by the at least one ultrasound transducer). The real-time feedback may comprise physiological data dynamically acquired during the stimulation session and indicative of the subject's ongoing neural state. The adaptation may be performed during stimulation to dynamically refine the control of the ultrasound field based on the subject's ongoing physiological response. Preferably, adapting the ultrasound stimulation parameters or the control signals may comprise dynamically adjusting the frequency and/or the phase of the ultrasound signals based on the real-time feedback during stimulation. Such dynamic adjustment may allow the system to optimize the delivery of ultrasound energy to the target brain region in response to changes in the subject's neural state or any detected physiological change, thereby improving targeting accuracy and enhancing neuromodulation efficacy.

In some embodiments, the physiological measurement system may be configured to monitor a frequency and/or a phase of a neuronal activity pattern in the subject. The neuronal activity pattern may comprise rhythmic or oscillatory neural signals (i.e. within s selected brain region) associated with ongoing brain activity, such as brain rhythms, spontaneous oscillations, or evoked potentials. In some embodiments, the control unit may be configured to generate control signals for transmitting an ultrasound waveform (i.e. with the at least one ultrasound transducer) that is phase-locked or frequency-locked to the neuronal activity pattern. Synchronizing the ultrasound waveform with the monitored neuronal activity may allow the stimulation to reinforce or modulate the natural dynamics of the brain, thereby improving the efficacy, selectivity, and reliability of neuromodulation.

In some embodiments, the control unit may be configured to generate test control signals for the at least one ultrasound transducer to transmit test ultrasound waveforms. The test ultrasound waveforms may be used to probe the target brain region and assess the subject's physiological response to controlled stimulation events. In some embodiments, the control unit may be further configured to refine the ultrasound stimulation parameters based on the physiological response monitored by the physiological measurement system to the test ultrasound waveforms. Such refinement may enable the system to optimize stimulation efficacy by dynamically calibrating the ultrasound parameters to match the subject's individual anatomical and physiological characteristics. The test ultrasound waveforms may correspond to ultrasound signals specifically designed for probing purposes, typically delivered at lower intensity or different timing configurations compared to therapeutic stimulation waveforms.

In some embodiments, the control unit may be configured to determine the ultrasound stimulation parameters based on brain structure arrangements, orientation of axons or white matter tracts within the target brain region, preferably,

such that the ultrasound radiation force is aligned with or orthogonal to the orientation of axons or white matter tracts. The orientation of axons or white matter tracts may be determined based on subject-specific information, such as diffusion tensor imaging (DTI) and/or magnetic resonance imaging (MRI) acquired from the subject, and/or based on predetermined anatomical information stored in a database accessible to the control unit. The database may be integrated into the application executed by the control unit and/or hosted externally and connected to the control unit. By aligning the ultrasound field or optimizing stimulation parameters in relation to the anatomical orientation of neural structures, the system may enhance the efficacy of mechanical stimulation and improve the specificity of neuromodulation within the targeted brain region.

In some embodiments, the control unit may be configured to determine the ultrasound stimulation parameters so as to induce strain in a cell body anchored to a dendritic tree within the target brain region. The stimulation parameters may be selected to generate mechanical forces that preferentially stretch the neuronal soma relative to its dendritic anchoring points. The control unit may determine the ultrasound stimulation parameters based on anatomical orientation information within the target brain region, assuming typical anchoring patterns of neuronal cell bodies and dendritic trees aligned with local tissue architecture. The system may optimize mechanical forces relative to known or inferred orientations of axonal fibers or cortical structures, thereby enhancing the probability of inducing strain in anchored cell bodies without requiring microscopic resolution of individual neuronal structures. By inducing strain in the anchored cell bodies, the system may enhance the likelihood of opening mechanosensitive ion channels, thereby facilitating modulation of neuronal excitability and improving the efficacy of neuromodulation. A high numerical aperture (NA), corresponding to a low F-number, may be preferred to achieve strong localized strain.

In some embodiments, the control unit may be configured to determine the ultrasound stimulation parameters to compensate for skull-induced aberrations based on anatomical data and/or on feedback from the physiological measurement system. The anatomical data may comprise structural information describing the morphology, thickness, or density variations of the subject's skull. Such anatomical data may correspond to information acquired for the particular subject, such as imaging data acquired (e.g., before and/or during operation of the system) from computed tomography (CT) or magnetic resonance imaging (MRI) or extracted by the physiological measurement system, and/or may correspond to generic information derived from a database comprising anatomical information of a plurality of subjects. Preferably, the feedback from the physiological measurement system may include information indicative of ultrasound field distortions during transmission through the skull. By compensating for skull-induced aberrations, the system may improve the spatial precision, intensity control, and overall efficacy of the ultrasound stimulation delivered to the target brain region.

In some embodiments, the control unit may be configured to determine the ultrasound stimulation parameters so as to optimize a spatial pressure gradient of the ultrasound field generated by the at least one ultrasound transducer. The spatial pressure gradient may refer to controlled variations in the acoustic pressure across the targeted brain region, influencing the mechanical strain induced within the tissue. In some embodiments, the optimization of the spatial pressure gradient may be achieved by controlling an apodization function applied to elements of the ultrasound transducer. The apodization function may refer to a spatial variation of the amplitude of the driving signals applied to different elements of the ultrasound transducer, thereby enabling control over the shape and distribution of the generated acoustic field. The apodization function may form part of the ultrasound stimulation parameters or the control signals generated by the control unit to drive the at least one ultrasound transducer. Preferably, the optimization of the spatial pressure gradient may be performed in one or more spatial directions selected from a lateral or medial direction (x-axis), an anterior or posterior direction (y-axis), and a superior or inferior direction (z-axis), thereby enabling directional control of the mechanical effects induced by the ultrasound stimulation.

In some embodiments, the control unit may be configured to optimize the apodization so as to maximize a ratio between a spatial derivative of acoustic pressure within the ultrasound field and a maximum acoustic pressure within the ultrasound field. The spatial derivative of acoustic pressure may refer to the rate at which the acoustic pressure changes across spatial coordinates within the brain tissue, indicative of the pressure gradients responsible for inducing mechanical strain. Preferably, the maximization may be based on a selected ratio from among:

-   M3, defined as: $$M3 = \left[\left(\frac{\partial P}{\partial x}\right)^2 + \left(\frac{\partial P}{\partial y}\right)^2 + (1/c)\left(\frac{\partial P}{\partial t}\right)^2\right] / P_{max}^2$$

-   M2, defined as: $$M2 = \left[\left(\frac{\partial P}{\partial x}\right)^2 + \left(\frac{\partial P}{\partial y}\right)^2\right] / P_{max}^2 \quad ; \text{ and}$$

-   M1, defined as: $$M1 = \left[\left(\frac{\partial P}{\partial x}\right)^2\right] / P_{max}^2$$

Wherein: P represents the acoustic pressure within the ultrasound field, $P_{max}$ is the maximum pressure, and x, y, and t represent the three space coordinates where "x" is the lateral or medial direction, "y" is the anterior or posterior direction, and "t" is proportional to the superior or inferior direction. In ultrasound, the depth (superior or inferior) coordinate z is proportional to time via z=ct, where c is the sound speed in tissue. Therefore, the $\partial t$ derivative can replace $\partial z$. System 100 can optimize pressure gradient metric M3 in all three directions (x, y, z), metric M2 in two directions (x, y), or metric M1 one direction (x). This allows System 100 to appropriately control the direction in which the neurons, axons, or tracts move.

M3, defined as the sum of the squared spatial pressure derivatives along lateral (x) and anterior-posterior (y) directions, and the squared temporal pressure derivative scaled by the sound speed (c), divided by the square of the maximum acoustic pressure (Pmax); M2, defined as the sum of the squared spatial pressure derivatives along x and y directions divided by $Pmax^2$; and M1, defined as the squared spatial pressure derivative along the x direction divided by $Pmax^2$.

In these definitions, P represents the acoustic pressure within the ultrasound field, x, y, and z represent spatial coordinates, c represents the sound speed in brain tissue, and Pmax represents the maximum amplitude of the acoustic pressure within the ultrasound field.

In some embodiments, the system may comprise at least two ultrasound transducers. The control unit may be configured to determine ultrasound stimulation parameters for the at least two ultrasound transducers so as to coordinate the ultrasound fields generated by the at least two ultrasound transducers within the target brain region. The coordination of the ultrasound fields may involve adjusting parameters such as focal depth, pressure amplitude, waveform timing, and field orientation to achieve a combined mechanical effect within the targeted brain tissue. By coordinating multiple ultrasound fields, the system may improve spatial targeting precision, enhance the distribution of mechanical strain, and enable more effective and selective neuromodulation of the target brain region.

In some embodiments, the control unit may be configured to determine the ultrasound stimulation parameters, the placement, and the angular pose of each of the at least two ultrasound transducers so that the at least two ultrasound transducers generate ultrasound fields directed substantially antiparallel to each other across the target brain region. The antiparallel fields may apply opposing acoustic radiation forces within the targeted region, thereby enhancing mechanical effects within the tissue.

In some embodiments, the ultrasound fields may have focal depths positioned short within the target brain region so as to form a limited non-overlapping space between the focal points of the two ultrasound fields. A limited non-overlapping space may refer to a region between focal points where the ultrasound fields are proximate but not overlapping, allowing the controlled formation of spatially confined standing waves. The control unit may be configured to selectively adjust the ultrasound stimulation parameters such that the ultrasound fields intersect with each other within the limited non-overlapping space, thereby causing spatially limited standing waves to form within the target brain region between the respective focal points of the two ultrasound fields. Generating spatially limited standing waves may enhance the localization and intensity of mechanical stimulation within a confined region of brain tissue.

Alternatively or additionally, the ultrasound fields may have focal depths configured to provide focal points arranged beyond each other within the target brain region so as to form a limited overlapping space between the focal points. A limited overlapping space may refer to a region where the focal points of the ultrasound fields intersect, enabling targeted antiparallel stretching of the tissue. The ultrasound fields may intersect with each other in the limited overlapping space, thereby causing antiparallel stretching of the target brain region. In some embodiments, the control unit may be configured to cause the at least two ultrasound transducers to operate at different ultrasound frequencies and/or to vary their angular pose in order to suppress the formation of standing waves within the target brain region. Suppressing standing waves in this configuration may allow for more uniform strain distribution across the target region and improve the efficacy of mechanical neuromodulation.

In some embodiments, the control unit may be configured to control at least one ultrasound transducer to alternately or simultaneously apply ultrasound fields directed, preferably substantially along a same main direction, to different sides of a single target brain region. The applied ultrasound fields may be configured to cyclically and/or continuously induce lateral shearing within the target brain region. Lateral shearing may refer to the relative displacement of adjacent tissue regions in directions parallel to the main axis of ultrasound field propagation, thereby generating mechanical strain that differs from pure compression or extension. By inducing controlled lateral shearing, the system may achieve enhanced modulation of neuronal structures within the target region, potentially improving the specificity and efficacy of the neuromodulation.

In some embodiments, the control unit may be configured to control the at least one ultrasound transducer to generate a collinear ultrasound radiation force directed along a series of anatomically connected regions within the target brain region. The anatomically connected regions may include structural pathways such as white matter tracts, neural fibre bundles, or sequential cortical areas. The anatomical information about the series of anatomically connected regions may correspond to subject-specific data, such as tractography or anatomical models derived from imaging data acquired (e.g., before and/or during operation of the system) from magnetic resonance imaging (MRI), diffusion tensor imaging (DTI), or may correspond to generic anatomical connectivity information derived from a database comprising anatomical models of a plurality of subjects. The control unit may be configured to align the ultrasound radiation force collinearly with the anatomical orientation of the connected regions based on this anatomical information, thereby improving the targeting accuracy and efficacy of mechanical strain induction. By applying a directed mechanical force along structurally connected pathways, the system may achieve selective modulation of targeted regions within the brain, thereby enhancing the precision and efficacy of neuromodulation.

Although the preceding description has referred primarily to a transcranial ultrasound stimulation system, the invention may also relate, according to a further aspect, to a method, such as a computer-implemented method, for controlling a transcranial ultrasound stimulation system. The method may correspond to or be derived from the functionalities and features described above with reference to the system. In particular, features described in relation to system components or their functionalities may be correspondingly expressed in terms of method steps performed by one or more processors or functional units of the system.

Thus, a second aspect of the invention refers to a method (e.g. a computer-implemented method) for controlling a transcranial ultrasound stimulation system, which is preferably a system according to any of the preceding embodiments of the first aspect of the invention.

The method generally corresponds to the functionalities and operations described in connection with the system. In particular, the method comprises receiving an input (e.g. by a control unit and preferably via a user interface connected to the control unit) indicating a selected stimulation objective and a target brain region for receiving the stimulation; monitoring, with a physiological measurement system, real-time physiological data from the subject; determining ultrasound stimulation parameters for at least one ultrasound transducer based on anatomical data associated with the selected target brain region and the selected stimulation objective, the ultrasound stimulation parameters preferably comprising at least one of a focal depth, a pressure to be applied, and stimulation timing; determining a placement and an angular pose of the at least one ultrasound transducer, and outputting the determined placement and angular pose to a user via a user interface; generating and providing guidance information for arranging the at least one ultrasound transducer at the determined placement and angular pose based on the real-time physiological data; and generating control signals for the at least one ultrasound transducer to transmit an ultrasound field adapted to apply an ultrasound radiation force within the target brain region based on the ultrasound stimulation parameters.

A third aspect of the invention refers to a computer program comprising instructions which, when executed by a control unit of a transcranial ultrasound stimulation system (e.g. preferably a system according to any embodiment of the first aspect of the invention), cause the transcranial ultrasound stimulation system to perform the method according to the second aspect of the invention. In some embodiments, the control unit may comprise the computer program configured to be executed on a processor configured to control stimulation parameters based on physiological and anatomical input. The control unit may be part of a transcranial ultrasound stimulation system, or may be implemented in another suitable processing device configured to control the system. The computer program may be embodied in a non-transitory computer-readable medium or may be accessible from a remote source.

## BRIEF DESCRIPTION OF THE DRAWINGS

Various embodiments of the invention will now be described, by way of example only, with reference to the accompanying drawings, in which:

Fig. 1A is a schematic block diagram illustrating an example implementation of a transcranial ultrasound stimulation system 100.

Fig. 1B shows an example of the transcranial ultrasound stimulation 100 system applied to a subject 105.

Fig. 2 illustrates example ultrasound waveforms generated by a transcranial ultrasound stimulation system 100.

Fig. 3A and Fig. 3B illustrate the effects of ultrasound waveforms with a low F-number and a high F-number, respectively.

Fig. 4A and Fig. 4B illustrate examples of stretching along an axon or a white matter tract generated by the ultrasound stimulation system 100.

Fig. 5 illustrates stretching of a neuronal cell body anchored to a dendritic tree using an ultrasound stimulation system 100.

Fig. 6 shows an example of the ultrasound stimulation system 100 generating orthogonal stretching relative to an axon.

Fig. 7A illustrates the use of antiparallel, spatially limited standing waves for neuromodulation with an ultrasound stimulation system 100.

Fig. 7B presents simulation graphs of antiparallel spatially limited standing waves.

Fig. 8A and Fig. 8B illustrate examples of antiparallel stretching generated by an ultrasound stimulation system 100.

Fig. 9A to Fig. 9E show examples of stretching and strain generated within a single brain region by an ultrasound stimulation system 100.

Fig. 10 shows an example of targeting tracts leading to or from deeper brain regions.

Fig. 11 illustrates collinear targeting of two brain regions.

Fig. 12 shows an example flowchart of a method for controlling transcranial ultrasound stimulation, preferably with an ultrasound stimulation system 100.

Fig. 13A to Fig. 13E illustrate example simulation results showing the effect of apodization on pressure gradient metrics.

It should be noted that the drawings are schematic only, are not necessarily to scale, and are provided for the purpose of illustrating selected embodiments of the invention, and do not represent anatomical accuracy

DETAILED DESCRIPTION OF THE DRAWINGS

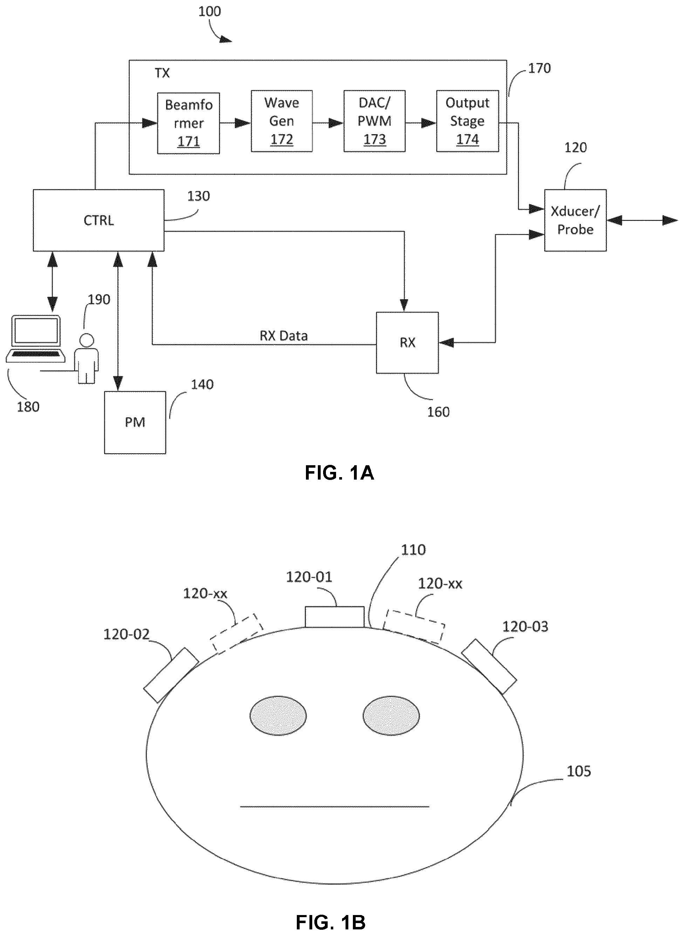

Fig. 1A schematically illustrates an example implementation of a transcranial ultrasound stimulation system 100 according to an embodiment of the invention. The system (100) comprises at least one ultrasound transducer 120, a physiological measurement system 140, a control unit 130, and a user interface 180.

The control unit 130 comprises at least one processor (e.g. at least one CPU, at least one AI processor, and/or at least one GPU) and an application (e.g. computer program or software solution) comprising instructions which, when executed by the at least one processor, control the operation of the system 100 as described herein. The control unit is further configured to determine ultrasound stimulation parameters for the at least one ultrasound transducer based on anatomical data associated with the selected target brain region and the selected stimulation objective. The anatomical data associated with the selected target brain region may comprise imaging data, anatomical models, or structural information describing the morphology or spatial properties of the brain. In some embodiments, the anatomical data may further correspond to predetermined information associating brain regions with suitable stimulation parameters for achieving predetermined stimulation objectives. Such predetermined information may be made available to the application, for example by being stored in the control unit, included as part of the application, or remotely accessible by the control unit. Further, the control unit 130 is exemplary represented as comprising (or being connected to) a transmit (TX) block 170, and a receive (RX) block 160.

The transmit block 170 is configured to manage the generation of ultrasound waveforms and may include a beamformer 171 (e.g. for generating beamforming parameters based on the ultrasound stimulation parameters determined by the control unit 130), a waveform generator 172 (e.g. for generating waveforms), a digital-to-analog converters with Pulse Width Modulation DAC/PWM 173 (e.g. for performing digital to analog conversion to instruct the at least one ultrasound transducer 120), and an output stage 174 for driving the ultrasound transducer element(s) 120 with the control signals. The receive block 160 may be configured to manage the reception of ultrasound signals from the at least one ultrasound transducer 120 and may include electronics as AFE (e.g. analog front-end, beamforming circuits, and signal processing units for reconstructing anatomical images of the brain tissue or for measuring real-time feedback).

The physiological measurement system 140 is configured to monitor physiological data from the subject 105, such as electroencephalography (EEG), magnetoencephalography (MEG), electromyography (EMG) signals, evoked potentials, or anatomical features acquired via ultrasound imaging (e.g. captured by the at least one ultrasound transducer). The physiological data may also be used to dynamically adapt the operation of the system in real-time. The control unit 130 may be further configured to generate guidance information for arranging the at least one ultrasound transducer at the determined placement and angular pose based on real-time physiological data monitored by the physiological measurement system.

The system 100 may comprise a plurality of ultrasound transducers (e.g., transducers 120-01, 120-02, 120-03 in Fig. 1B), which may be single-element transducers or array transducers (e.g. each ultrasound transducer 120 comprising a plurality of transducer elements). The system 100 (e.g. the control unit 130) may automatically suggest the number of transducers to be used, the placement locations and angular poses of the transducers, and the ultrasound stimulation parameters, based on the selected stimulation objectives and target brain regions. The ultrasound stimulation parameters may include one or more of a focal depth, a pressure to be applied, stimulation timing, or characteristics of the ultrasound waveform (e.g. frequency, amplitude, etc.). The ultrasound transducers 120 may be used for stimulation, imaging, or both, and real-time imaging from the transducers may be used to adjust the timing or parameters of stimulation.

The control unit 130 may be configured to determine the ultrasound stimulation parameters based on an orientation of axons or white matter tracts within the target brain region. The orientation of axons or white matter tracts may be determined based on subject-specific information, such as diffusion tensor imaging (DTI) and/or magnetic resonance imaging (MRI) acquired from the subject, and/or based on predetermined anatomical information stored in a database accessible to the control unit. The database may be integrated into the application executed by the control unit and/or hosted externally and connected to the control unit. By aligning the ultrasound field or optimizing stimulation parameters in relation to the anatomical orientation of neural structures, the system may enhance the efficacy of mechanical stimulation and improve the specificity of neuromodulation within the targeted brain region.

The system 100 (e.g. the control unit 130) may receive (e.g. via the physiological measurement system 140 or via one or more auxiliary measurement systems - e.g. configured to provide real-time imaging) input anatomical data derived from imaging modalities such as magnetic resonance imaging (MRI) or computed tomography (CT) scans, and may perform simulations to model ultrasound field propagation through the subject's skull and brain tissue. Such simulations may allow the system 100 (e.g. the control unit 130) to account for individual anatomical features, such as skull thickness variations, bone curvature, axon and/or white matter tract orientations, and the presence of hair, in determining the optimal transducer positioning and ultrasound parameters. Real-time imaging and physiological measurement data may further enable the system to refine the ultrasound field characteristics dynamically.

The system 100 may be further configured to simulate the transformation of ultrasound fields due to the skull and perform phase corrections to compensate for aberrations caused by refraction or field distortion. Simulated results, including predicted field distributions and mechanical strain patterns, may be presented to the clinician 190 via the user interface 180 to assist in the placement and operation of the transducers. The control unit 130 may optimize ultrasound stimulation parameters, including the spatial pressure gradient, focal depth, pressure amplitude, and waveform timing, in order to maximize mechanical effects such as targeted stretching of neuronal structures.

The system 100 may operate manually, semi-automatically, or in a combination thereof, allowing the clinician 190 to retain control while benefiting from system-assisted guidance and real-time optimization based on feedback data (e.g. as provided by the physiological measurement system 140). The control unit 130 may coordinate the operation of multiple transducers, potentially with phase offsets or different frequencies, to achieve more effective stimulation patterns, including cyclic stretching, antiparallel force generation, or suppression of undesired standing wave formations.

Even though the clinician 190 may have selected a single target region, the control unit 130 may suggest applying the ultrasound field to one or more target regions. For instance, the system 100 may suggest aiming at two sides of the target region alternately, causing cyclic stretching between the left and right sides of the target. This can be done using a single transducer 120 or more than one transducer 120.

The user interface 180 enables a user, such as a clinician 190, to interact with the system 100, for instance by selecting target brain regions, entering stimulation objectives, reviewing system-suggested placements of the transducer(s) 120, and monitoring real-time data. The user interface 180 may display anatomical images, physiological measurement data, guidance information and/or simulation results showing predicted ultrasound fields and mechanical effects.

In some embodiments, the system 100 may use subject-specific anatomical data, such as MRI acquired prior to the procedure, or generic brain atlas data, to perform simulations (e.g. of anatomical constraints) for guiding neuromodulation. Real-time imaging data, for example from the receive functionality of ultrasound transducers 120, may additionally be used to refine the simulations and guidance. The clinician 190 may be enabled to modify stimulation parameters such as focal depth, field direction, correction parameters, ultrasound pressure or pressure gradients via the user interface 180, based on visualized simulation output or measured feedback. The system 100 and control unit 130 may simulate ultrasound field distortions caused by subject-specific skull anatomy and apply phase corrections to mitigate aberrations introduced by refraction or reflection at the bone interface. For example, in order to achieve a high numerical aperture (NA) field capable of producing high focusing strength for providing strong mechanical strain within the brain tissue, the system may need to adjust the control signals to account for skull-induced deviations. The skull may alter the pressure field shape by causing lateral refraction, focus shortening, or lensing effects. These effects may lead to spatial displacements of the intended stimulation target. By incorporating high-resolution simulations of skull-bone interaction, the system 100 may enhance the accuracy of ultrasound field positioning and maximize the efficacy of neuromodulation.

Preferably, the control unit 130 may be configured to determine the ultrasound stimulation parameters so as to optimize a spatial pressure gradient of the ultrasound field generated by the at least one ultrasound transducer 120. The spatial pressure gradient may refer to controlled variations in the acoustic pressure across the targeted brain region, influencing the mechanical strain induced within the tissue. In some embodiments, the optimization of the spatial pressure gradient may be achieved by controlling an apodization function applied to elements of the ultrasound transducer. The control unit may be configured to optimize the apodization so as to maximize a ratio between a spatial derivative of acoustic pressure within the ultrasound field and a maximum acoustic pressure within the ultrasound field. Preferably, the maximization may be based on a selected ratio from among one of the previously described ratios M1, M2 and M3.

Ultrasound timing

The control unit 130 may be further configured to adapt the ultrasound stimulation parameters or the control signals based on real-time feedback derived from physiological data monitored by the physiological measurement system (e.g. by electroencephalography (EEG), magnetoencephalography (MEG), electromyography (EMG) signals, or imaging data captured by the at least one ultrasound transducer). The real-time feedback may comprise physiological data dynamically acquired during the stimulation session and indicative of the subject's ongoing neural state. The adaptation may be performed during stimulation to dynamically refine the control of the ultrasound field based on the subject's ongoing physiological response. Preferably, adapting the ultrasound stimulation parameters or the control signals may comprise dynamically adjusting the frequency and/or the phase of the ultrasound signals based on the real-time feedback during stimulation. Such dynamic adjustment may allow the system to optimize the delivery of ultrasound energy to the target brain region in response to changes in the subject's neural state or any detected physiological change, thereby improving targeting accuracy and enhancing neuromodulation efficacy.

Neuronal activity has a natural frequency. For example, Theta waves have a frequency range of 4-8 Hz and are associated with the oscillation used by the hippocampus to connect to other brain circuits. Table 1 below lists the time scales of neuronal activity and its significance.

*Table 1*

| Period | Frequency Band | Biological Significance |
|---|---|---|
| 100 s~100+ min | <0.01Hz | Longer-term plasticity mechanisms |
| 100 ms~100 s | 0.01~10 Hz | Local shorter-term plasticity |
| 250~1000 ms | 1~4 Hz | Delta |
| 125~250 ms | 4~8 Hz | Theta |
| 80~125 ms | 8~12.5 Hz | Alpha |
| 40~80 ms | 12.5-25 Hz | Beta |
| 12.5~40 ms | 25~80 Hz | Low gamma |
| 5~12.5 ms | 80~200 Hz | High gamma |
| 0.1~5 ms | 200~10000 Hz | Membrane time constant, AP (action potential) width, max spike firing rates |
| Beyond | Beyond | Likely smeared by membrane time constants |

In some embodiments, the control unit 130 may be configured to generate ultrasound waveforms whose timing parameters are selected based on the natural oscillation characteristics of the target brain region. Use of timing parameters consistent with a brain region's natural response can reduce the total time for achieving the stimulation objective compared to systems that rely on a more trial- and-error-based approach for the ultrasound waveform parameters, similar to pushing a swing at the right time, in phase and at the natural oscillation frequency.

Thus, the physiological measurement system may be configured to monitor a frequency and/or a phase of a neuronal activity pattern associated with ongoing brain activity. Preferably, the control unit may be configured to generate control signals for transmitting an ultrasound waveform (i.e. with the at least one ultrasound transducer) that are phase-locked or frequency-locked to the neuronal activity pattern. By generating waveforms that are phase-locked to the neuronal activity within the selected target region, the system 100 may enhance the coupling efficiency between the ultrasound stimulation and the neuronal oscillations, thereby improving the efficacy of neuromodulation. In some cases, the waveforms may be configured to coincide with peaks of neuronal excitability to maximize neural activity, or alternatively may be configured to inhibit activity by generating waveforms that are contrary to neuronal oscillation (e.g. out of phase, constant or at frequencies remote from the natural oscillation frequency of the neuronal structures). The system 100 may dynamically adjust the frequency and phase of the generated waveforms based on real-time physiological feedback acquired, for example, via the physiological measurement system 140, including EEG signals, evoked potentials, or ultrasound imaging data.

Furthermore, in some embodiments, the control unit 130 may generate layered or combined waveforms using amplitude modulation, gating, or triggering techniques. Combined waveforms may involve frequencies or phases from two or more neuronal frequency bands (e.g. as those listed in Table 1) and may be adjusted in their duty cycle or phase to optimize the modulation of neuronal activity patterns. By enabling dynamic adaptation and combination of waveform characteristics, the system 100 may more precisely tailor the ultrasound stimulation to the ongoing physiological state of the subject and to the desired stimulation objective.

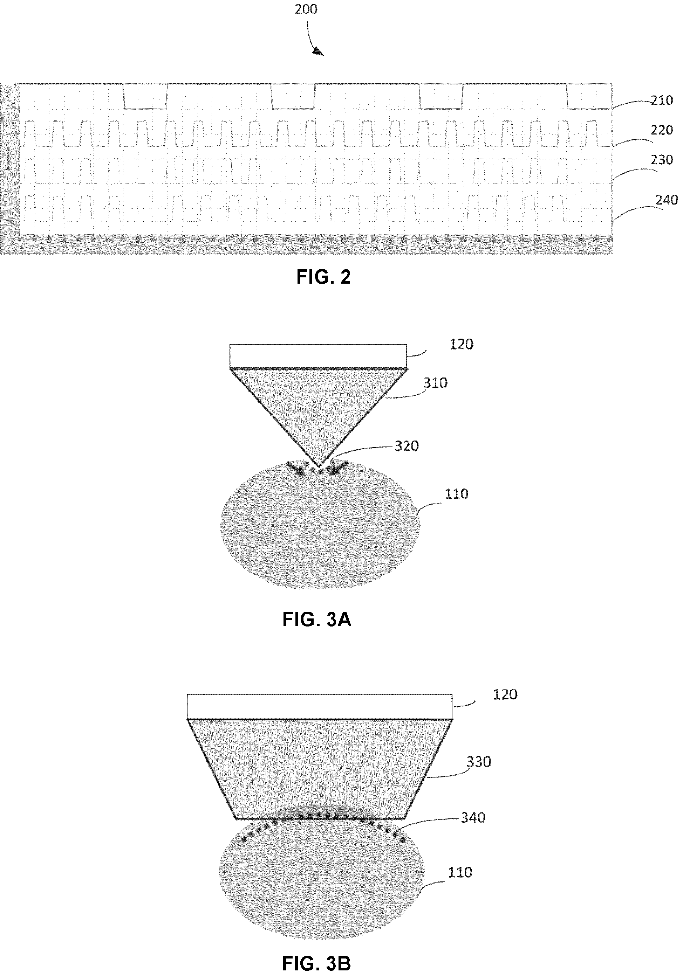

Fig. 2 illustrates example ultrasound waveforms that may be generated by a transcranial ultrasound stimulation system 100 according to an embodiment of the invention. Waveform 210 represents a slow oscillation waveform having an exemplary period of approximately 100 milliseconds and a duty cycle of approximately 70%, corresponding to an ON-time of approximately 70 milliseconds. Waveform 220 represents a fast oscillation waveform having a period of approximately 19 milliseconds and a duty cycle of approximately 31%, corresponding to an ON-time of approximately 6 milliseconds, and including an offset of approximately 4 milliseconds relative to waveform 210.

Waveform 230 illustrates a gating waveform, obtained by applying a logical "AND" operation between waveform 210 and waveform 220. In this example, waveform 230 has the same phase as waveform 220. Waveform 240 illustrates a triggering waveform example, which results when waveform 210 triggers the generation of waveform 220. In waveform 240, the fast oscillation begins when waveform 210 turns on, but with an offset of approximately 4 milliseconds. The second and third bursts of waveform 230 and waveform 240 highlight the differences between the gating and triggering operations. These waveform combinations enable the system 100 to flexibly modulate ultrasound delivery patterns based on desired neuronal stimulation protocols.

Ultrasound waveforms and filed shape

Figs. 3A and 3B illustrate the effects of ultrasound waveforms generated with different focusing characteristics (e.g. determined by the control unit 130 as part of the ultrasound stimulation parameters). The use of a weakly focused waveform, as shown in Fig. 3B, may lead to compression of the tissue. However, such changes are generally not optimal for enacting neural activity at safe or reasonable pressures. In contrast, optimally configured ultrasound waveforms and fields without requiring high peak intensities and pressures can effectively stretch neurons, thereby opening ion channels or collapsing neurotransmitter vesicles into the presynaptic terminal membrane, leading to changes in neural activity or communication.

The focusing strength of an ultrasound beam can be expressed as an F-number or a numerical aperture (NA). The following equation relates NA and F-number:

$$NA = (\frac{1}{2F})$$

Fig. 3A shows an example of a low F-number field (corresponding to a high NA or a tightly focused field). An ultrasound transducer 120 of the transcranial ultrasound stimulation system 100 generates an ultrasound field 310, which stretches the shallow cortex of the subject's head 110, resulting in strain 320.

Fig. 3B shows an example of a high F-number field. In this case, the transducer 120 generates an ultrasound field 330, and the resulting effect is illustrated as displacement 340. In both examples, the applied acoustic power is the same; however, the lower F-number configuration in Fig. 3A creates greater strain because the displacement of tissue is more spatially confined relative to the surrounding tissue.

High F-number fields are less effective in enacting changes in neuronal activity. Fields generated using a high F-number tend to move a broad region downward or cause compression of the tissue. In the shallow cortex, especially close to the ultrasound transducer, there are relatively few mechanisms available to enact neuronal stretching. While local mechanisms relying solely on pressure or intensity (e.g., local membrane cavitation, jet streaming) have been proposed as possible mechanisms for ultrasonic neuromodulation, negative results in large animals or in experimental settings where standing waves and other confounding factors are properly minimized suggest that these local mechanisms are not the predominant mechanisms responsible for strong effects such as evoked motor responses.

As transcranial magnetic stimulation (TMS) has demonstrated, even reliable and strong momentary stimulation does not easily lead to long-lasting stimulation effects, thereby highlighting the critical importance of obtaining strong, predictable, and reliable ultrasonic neuromodulation.

The focusing characteristics of the ultrasound field, and thus the effective F-number or numerical aperture (NA), may be controlled dynamically by the system 100 as part of the determination of the ultrasound stimulation parameters conducted by the control unit 130. For example, the control unit 130 may adjust the effective aperture of the ultrasound field by controlling the activation pattern, weighting, or timing of the elements of an array-type ultrasound transducer. The aperture may thus be considered one of the ultrasound stimulation parameters determined by the control unit 130, alongside other parameters such as, inter alia, focal depth, pressure amplitude, and stimulation timing. By dynamically adapting the aperture, the system 100 may modify the spatial confinement and focusing strength of the ultrasound field to optimize the mechanical strain induced within the target brain region based on the anatomical characteristics of the subject and the selected stimulation objective.

Optimizing pressure spatial derivative or gradient

In some embodiments, the system 100 may be configured to generate ultrasound waveforms whose intensity (peak pressure) is selected to improve modulation strength or reliability. However, increasing peak pressure alone has limited efficacy, and regulatory or safety constraints typically limit the maximum negative pressure excursion and maximum power deposition into biological tissue.

The spatial pressure derivatives, or gradients, within the ultrasound field may vary independently of the absolute pressure magnitude. Increasing the center acoustic frequency of the stimulation waveform may increase the spatial gradient in the axial direction; however, the degree of frequency increase is constrained by attenuation effects in the skull, which limit the usable frequency range. Acoustic fields that exhibit higher spatial pressure derivatives are generally more efficacious for achieving neuromodulation. Fields lacking significant spatial pressure gradients may be ineffective in producing the mechanical strain mechanisms underlying ultrasonic neuromodulation.

Positive neuromodulation effects observed in clinical settings may sometimes result from the incidental exposure of a target structure to the "shoulder" regions of the ultrasound beam, where significant pressure gradients exist due to slight offsets of the focus relative to the structure. Similarly, if the target structure is larger than the ultrasound focal volume, parts of the structure may experience significant pressure gradients. A higher spatial pressure gradient translates to more effective stretching of neural tissues and thus more effective neuromodulation.

To enhance neuromodulation efficacy, the system 100 may determine and generate ultrasound waveforms, apodization functions, apertures, or combinations thereof, that maximize the spatial pressure gradient. In some embodiments, for a given treatment volume or target area, the system 100 may optimize the generated fields by maximizing one of several defined pressure gradient metrics, denoted M1, M2, and M3. These metrics are defined as follows:

As previously described, the control unit 130 may be configured to determine the ultrasound stimulation parameters so as to optimize a spatial pressure gradient of the ultrasound field generated by the at least one ultrasound transducer. In some embodiments, the optimization of the spatial pressure gradient may be achieved by controlling an apodization function applied to elements of the ultrasound transducer. Preferably, the optimization of the spatial pressure gradient may be performed in one or more spatial directions selected from a lateral or medial direction (x-axis), an anterior or posterior direction (y-axis), and a superior or inferior direction (z-axis), thereby enabling directional control of the mechanical effects induced by the ultrasound stimulation.

In some embodiments, the control unit may be configured to optimize the apodization function so as to maximize a ratio between a spatial derivative of acoustic pressure within the ultrasound field and a maximum acoustic pressure within the ultrasound field. The spatial derivative of acoustic pressure may refer to the rate at which the acoustic pressure changes across spatial coordinates within the brain tissue, indicative of the pressure gradients responsible for inducing mechanical strain. Preferably, the maximization may be based on a selected ratio from among:

- M3, defined as:

$$M3 = \left[\left(\frac{\partial P}{\partial x}\right)^2 + \left(\frac{\partial P}{\partial y}\right)^2 + (1/c)\left(\frac{\partial P}{\partial t}\right)^2\right]/P_{max}^2$$

- M2, defined as:

$$M2 = \left[\left(\frac{\partial P}{\partial x}\right)^2 + \left(\frac{\partial P}{\partial y}\right)^2\right]/P_{max}^2;$$

and

- M1, defined as:

$$M1 = \left[\left(\frac{\partial P}{\partial x}\right)^2\right]/P_{max}^2$$

Wherein: P represents the acoustic pressure within the ultrasound field, Pmax is the maximum pressure, and x, y, and t represent the three space coordinates where "x" is the lateral or medial direction, "y" is the anterior or posterior direction, and "t" is proportional to the superior or inferior direction. In ultrasound, the depth (superior or inferior) coordinate z is proportional to time via z=ct, where c is the sound speed in tissue. Therefore, the $\partial$t derivative can replace $\partial$z. System 100 can optimize pressure gradient metric M3 in all three directions (x, y, z), metric M2 in two directions (x, y), or metric M1 one direction (x). This allows System 100 to appropriately control the direction in which the neurons, axons, or tracts move.

These metrics may be normalized to the maximum pressure $P_{max}$ in order to make the metrics independent of the absolute pressure applied. For neuromodulation purposes, fields and waveforms that maximize these metrics are preferred so that the lowest possible pressure can be selected for treatment.

The system 100 (e.g. by means of the control unit 130) may optimize the apodization function - defined as the spatial variation in drive voltage amplitudes applied to the transducer elements by the control unit 130 (e.g. by means of the output stage 174) - to maximize these gradient metrics. In some embodiments, the system 100 may also utilize two or more apertures operating at different frequencies and generating intersecting ultrasound beams to further enhance spatial pressure gradients.

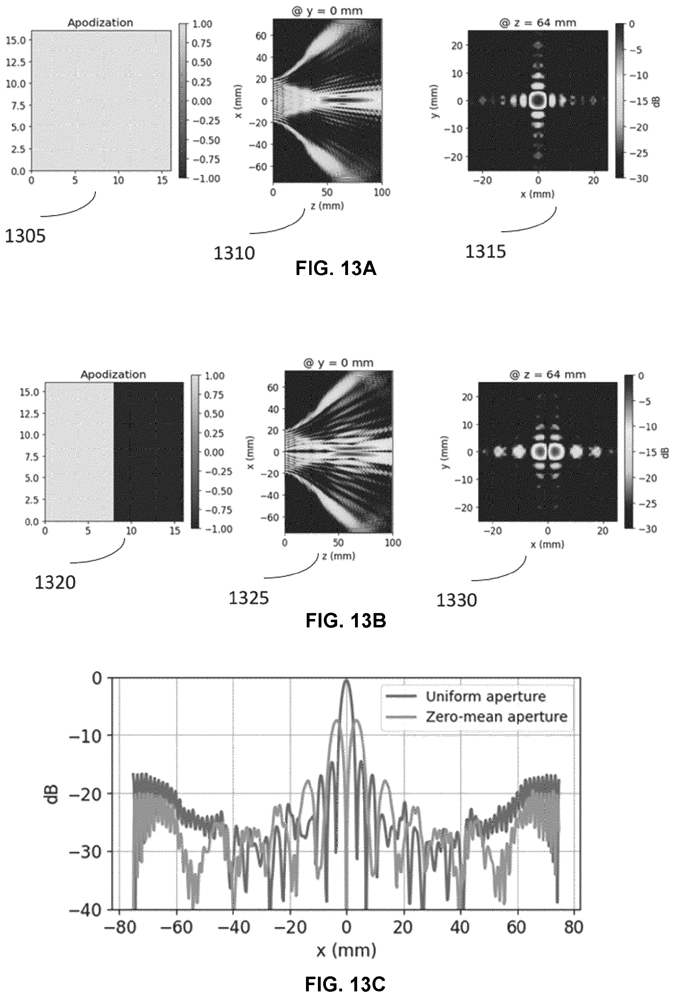

Fig. 13A-E shows examples of (simulation) results obtainable by a system 100 when the control unit 130 is configured to optimize the apodization function to maximize a ration between a spatial derivative of acoustic pressure and a maximum acoustic pressure within an ultrasound field. The quantity $P^2_{max}$ used in the definition of metrics M1, M2 and M3 is held constant. All these simulations are done at the same frequency of 0.75 MHz, which means that the value of $(\partial P/\partial t)^2$ in the formula for the metric M3 is the same in each simulation. These simulations only show improvements in the spatial metrics M1 and M2 when the apodization function is altered.

Fig. 13A (prior art) shows a simulation of a 16x16 element (exemplary and non-limiting configuration, since the number of elements may be generically defined as a plurality) ultrasound transducer. In this simulation, the apodization function 1305 is flat, meaning that all elements of the transducer are driven with uniform amplitude. The plot 1310 shows the acoustic field in depth ("z") and laterally ("x") with the "y" coordinate set to zero. A broadside focus can be seen as well as grating lobes around 45 degrees. Plot 1315 shows the acoustic field "x" and "y" directions at a fixed depth (z=64 mm). This example is a common way of operating an ultrasound system, where there is no attempt to maximize the pressure gradient. This produces neuromodulation as a side effect and is inefficient in its usage of ultrasonic power.

Fig. 13B shows an apodization function 1320 which improves the field gradient in the lateral or "x" direction. Plot 1330 shows a rapid pressure change at and near x=0 for y coordinates close to y=0. The apodization function shown in 1320 produces a better M1 metric than the function shown in Fig. 13A. Plot 1330 shows the acoustic field "x" and "y" directions at a fixed depth (z=64 mm).

Fig. 13C compares the pressure magnitudes as a function of lateral position (x-axis) between the configurations of Figs. 13A and 13B, showing that the spatial derivative $\partial P/\partial x$ is greater in the improved apodization case (Fig. 13B) near x = 0.

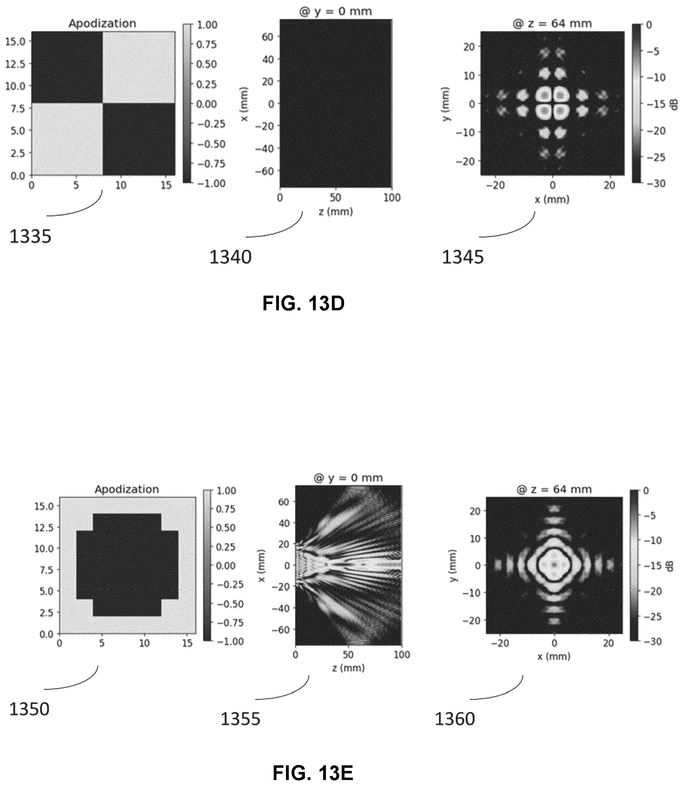

Fig. 13D shows a further example where an apodization function 1335 is used to enhance the spatial pressure derivatives in both the lateral (x) and anterior-posterior (y) directions, thereby improving metric M2. The resulting acoustic field 1340 shows reduced sound intensity along the y=0 plane, while plot 1345 illustrates the increased spatial derivatives.

Fig. 13E illustrates a different apodization function 1350 that also improves metric M2 by adjusting the pressure distribution in both lateral (x) and anterior-posterior directions (y), as shown in plots 1325 and 1330.

Anatomically driven transcranial ultrasound stimulation acoustic fields

In some embodiments, the control unit may be configured to determine the ultrasound stimulation parameters based on anatomical characteristics (e.g. based on brain structures arrangement, orientation of axons or white matter tracts within the target brain region). The orientation of axons or white matter tracts may be determined based on subject-specific information, such as diffusion tensor imaging (DTI) and/or magnetic resonance imaging (MRI) acquired from the subject, and/or based on predetermined anatomical information stored in a database accessible to the control unit. The database may be integrated into the application executed by the control unit and/or hosted externally and connected to the control unit.

Thus, the system 100 may be configured to generate (e.g. by means of the control unit 130 generating parameters to be applied via the at least one ultrasound transducer) treatment fields that are anatomically driven (e.g. taking into account the structural and functional relationships between brain regions).

Brain structures are often connected to other regions via axons and other structures. In addition, the extracellular matrix inherently holds together brain cells and structures locally. When these other regions are not being displaced by ultrasound, we can consider them "anchored" due to their surrounding structures. These structures may be more distal structures connected to the target region via axons. They may be the target brain region while the surrounding non-target region can be considered anchored. They may also be the periphery of the target brain region if the ultrasound field is highly focused and only applying significant displacement to a portion of the target region. Analyzing these brain regions or structures, it is evident that moving the brain regions toward a connected, anchored brain region creates some slack (and not strain), leading to less efficacious neural modulation. Anatomically driven TUS treatment fields consider this anchoring of brain structures relative to their surrounding regions or other brain regions. This means a correctly directed field produces a radiation force that creates efficacious strain within the cell bodies or processes such as axons and dendrites, thus leading to more efficacious neural modulation. The following sections provide examples of System 100 generating treatment fields driven by the target volume's anatomy

Stretching along axon or white matter tract

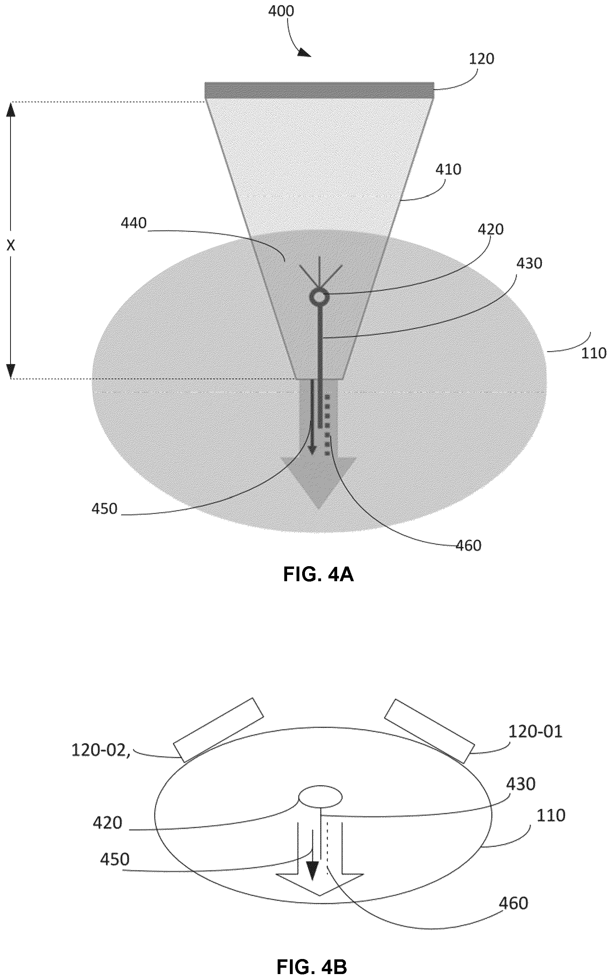

Figs. 4A and 4B illustrate examples of ultrasound stimulation fields generated by a transcranial ultrasound stimulation system 100, configured to induce mechanical strain within neural structures such as axons or white matter tracts based on their anatomical orientation.

Brain regions exhibit varying degrees of structural organization. In some cases, neural structures, such as axonal tracts or layers of neurons, are highly ordered in a predetermined orientation. Even within a single brain region, subpopulations of neurons, such as projection neurons, may exhibit ordered arrangements, whereas local interneurons may appear more disorganized.

In some embodiments, the control unit 130 may be configured to determine ultrasound stimulation parameters such that the radiation force generated by the ultrasound field is aligned with the orientation of axons or white matter tracts within the target brain region. The anatomical orientation information may be determined based on subject-specific imaging data, such as diffusion tensor imaging (DTI), and/or based on predetermined anatomical models accessible by the control unit 130.

Fig. 4A shows an schematic view 400 of an example where the system 100 generates an ultrasound field 410 using at least one ultrasound transducer. A neuron 420 is illustrated with an axon 430 extending from the cell body and dendritic structures 440. The ultrasound field 410 is focused such that the axial depth of focus (denoted as X) falls beyond the neuron 420 and within the axon 430. This configuration produces an acoustic radiation force (ARF), schematically indicated by the wider arrow, which is maximal within the axon 430 rather than in the soma (cell body). As a result, longitudinal strain 450 is induced along the axon 430, thereby stretching the neuronal structure as indicated by deformation 460.

To maximize strain within the axon relative to the displacement of surrounding or shallower structures, the system 100 may utilize a low F-number focus configuration (which is determined by the control unit 130). In white matter tracts, which are composed of aligned bundles of axons, this approach can similarly maximize mechanical strain along the tract direction, thereby enhancing the efficacy of neuromodulation.

Skull reflections and anatomical constraints may limit the extent to which the F-number can be reduced in practice. To address this, and to further enhance strain induction, Fig. 4B illustrates an embodiment where two ultrasound transducers 120-01 and 120-02 are positioned at different locations on the subject's head 110. These ultrasound transducers 120-01 and 120-02 are configured to cooperatively generate ultrasound fields (i.e. based on the determination of the ultrasound stimulation parameters by the control unit 130) that differentially displace structures 460 relative to anchored surrounding tissue, resulting in increased mechanical strain 450 within the target structure.

In some embodiments, the relative angular orientation between ultrasound transducers 120-01 and 120-02 may be dynamically adjusted based on anatomical information and stimulation objectives. More than two transducers may also be used to refine the generation of directional strain within complex neural structures.

Stretching orthogonal to dendritic tree

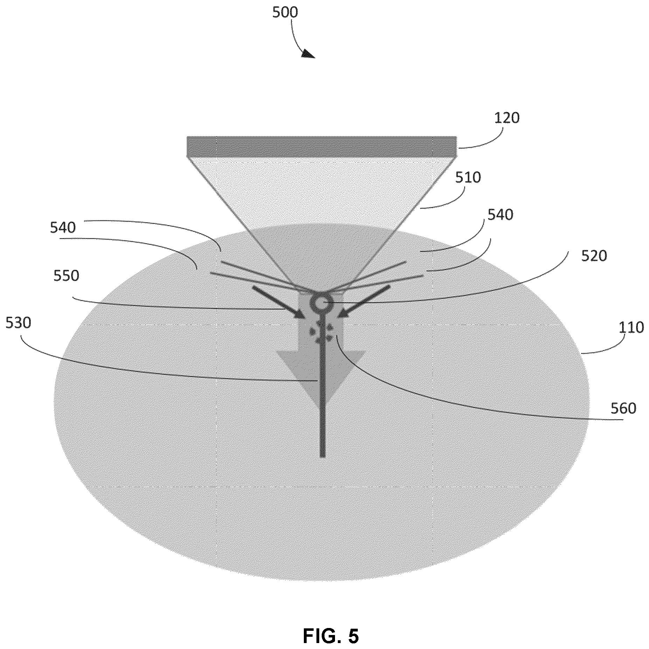

Fig. 5 illustrates an schematic view 500 of an example of a transcranial ultrasound stimulation field generated by a system 100, configured to induce mechanical strain within a neuronal structure comprising a cell body anchored to a dendritic tree, according to an embodiment of the invention.

In brain regions where dendrites extend laterally over a considerable distance and exhibit relatively ordered arrangements, these dendritic structures may function as an anchoring network for neuronal cell bodies. When dendrites are well anchored, such as in the case of a dendritic tree spanning multiple millimeters, a targeted displacement of the neuronal soma relative to the dendritic anchoring points can generate effective mechanical strain within the neuron.

Fig. 5 shows a neuron 520 with an axon 530 and a well-anchored dendritic tree 540. An ultrasound transducer 120 of the system 100 generates an ultrasound field 510, focused with an axial depth sufficient to center on the neuronal soma (cell body) 520. The acoustic radiation force (ARF) resulting from the ultrasound field is represented schematically by a wider arrow. This force preferentially displaces the cell body downward relative to the anchored dendritic tree 540, thereby inducing mechanical strain 550 and resulting in the deformation 560 of the neuronal structure.

In this embodiment, the system 100 (e.g. the control unit 130) may optimize the ultrasound stimulation parameters, including the focal depth, focal diameter, and field orientation, to preferentially stretch the neuronal soma relative to the dendritic structures. A high numerical aperture (NA), corresponding to a low F-number, may be preferred to achieve strong localized strain. If a low NA configuration ("flat wavefront") were used, the ultrasound field would tend to displace both the dendritic tree and the cell body together without producing significant differential strain, similar to the broad compression effects described previously in Fig. 3B.

By generating ultrasound fields that induce strain specifically in anchored neuronal cell bodies, the system 100 may enhance the probability of opening mechanosensitive ion channels, thereby facilitating modulation of neuronal excitability and improving the efficacy of neuromodulation.

Stretching orthogonal to axon or white matter tract

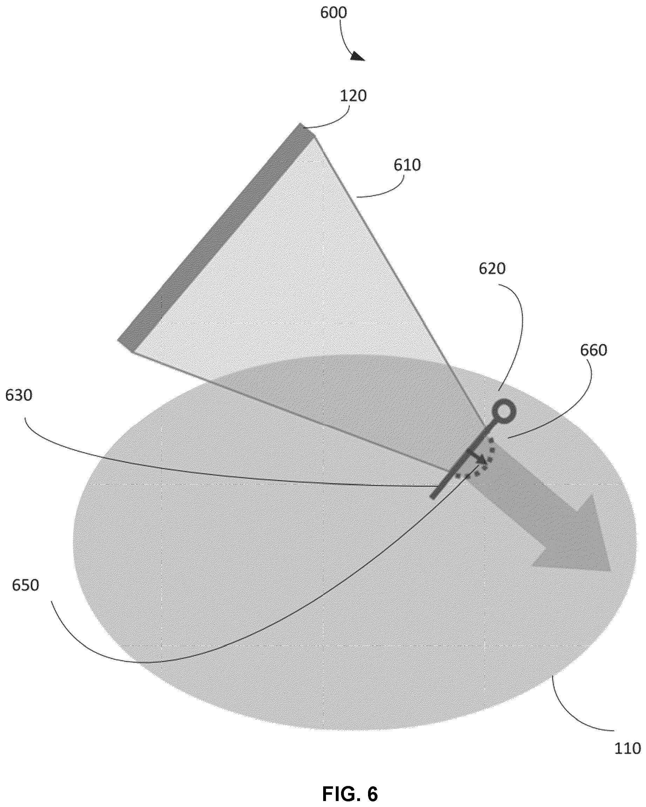

Fig. 6 illustrates an schematic view 600 of an embodiment of the at least one ultrasound transducer 120 of the transcranial ultrasound stimulation system 100 generating an ultrasound field configured to induce mechanical strain orthogonal to the orientation of an axon, according to an embodiment of the invention. This produces an effect similar to plucking a guitar string.

In this example, a neuron 620 is shown with an axon 630. An ultrasound field 610 is generated by the ultrasound transducer 120 of the system 100. The field 610 is oriented substantially orthogonally with respect to the longitudinal axis of the axon 630. This configuration produces an acoustic radiation force (ARF), schematically illustrated by a wider arrow, acting transverse to the axonal direction.

The resulting mechanical effect is a lateral deformation or bending of the axonal structure, thereby inducing strain 650, which is shown as displacement 660. This strain pattern may resemble the transverse displacement observed when plucking a stretched string, such as a guitar string, and may lead to modulation of neuronal activity via activation of mechanosensitive structures within or around the axon.

In some embodiments, the control unit 130 may determine the ultrasound stimulation parameters so as to control the direction and spatial confinement of the ultrasound field relative to the anatomical orientation of the neural structures within the target region. To this end, anatomical information describing the morphology and spatial orientation of axons or white matter tracts may be obtained from subject-specific data, such as diffusion tensor imaging (DTI), or retrieved from generic anatomical models.

Furthermore, to enhance spatial precision and compensate for field distortions caused by transmission through the skull, the control unit 130 may be configured to adapt the ultrasound stimulation parameters based on anatomical data and/or feedback from the physiological measurement system 140. The anatomical data may include information describing skull morphology, thickness, or density variations, obtained for example from computed tomography (CT), magnetic resonance imaging (MRI), or real-time imaging acquired by the system. Preferably, the feedback may include real-time physiological or acoustic measurements indicative of ultrasound field aberrations. By compensating for skull-induced aberrations, the system 100 may maintain precise targeting of neural structures and ensure the desired field orientation relative to the target, thereby improving the efficacy and reliability of orthogonally directed neuromodulation.

Antiparallel, spatially limited standing waves

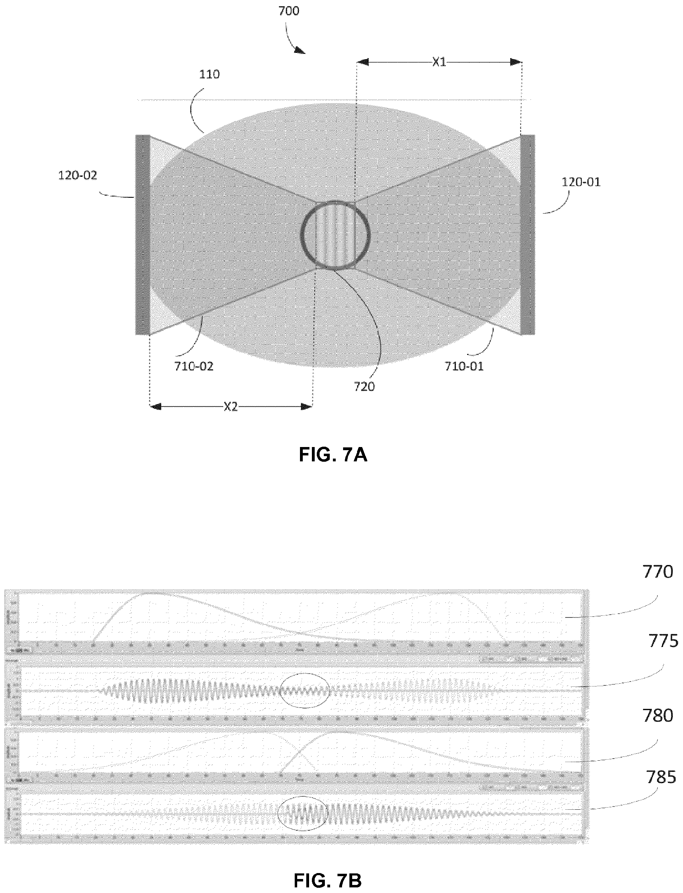

Figs. 7A and 7B illustrate examples of the generation of antiparallel ultrasound fields with spatially limited standing waves by a transcranial ultrasound stimulation system 100, according to embodiments of the invention.

In some embodiments, the control unit 130 may determine the placement, orientation (e.g. angular pose), and ultrasound stimulation parameters of at least two ultrasound transducers to direct their respective ultrasound fields substantially antiparallel across a target brain region. By appropriately selecting the focal depths and angular poses of the transducers, spatially confined standing waves can be formed within a limited region of the target.

Fig. 7A shows an schematic view 700 of a subject's head 110 with a target brain region depicted as circle 720. Two ultrasound transducers, 120-01 and 120-02, are positioned on opposite sides of the subject's head. The ultrasound fields 710-01 and 710-02 generated by the two transducers are directed toward each other in an antiparallel arrangement. The focal depths of the two fields, indicated as X1 and X2, are configured to aim slightly short relative to the exact target center, such that the standing wave formation is spatially limited to a controlled region within the target 720. This region is also referred to as limited non-overlapping space between the focal points (or focal regions) of the two ultrasound fields

In this configuration, spatially limited standing waves, illustrated as banded lines within the target region 720, are formed where the ultrasound pressure envelopes from the two fields overlap. The limited extent of the standing waves is achieved by controlling the focusing parameters (e.g., focal depth, F-number) and the relative positioning and angular orientation of the transducers. By limiting the spatial extent of the standing waves, the system 100 may enhance the localization of the mechanical stimulation effect while reducing off-target modulation/activation.

Fig. 7B provides simulation graphs illustrating the formation of the antiparallel spatially limited standing waves. The graphs represent simulated acoustic pressure fields under exemplary system 100 configurations. Graph 770 shows the pressure envelopes generated by transducers 120-01 and 120-02 as functions of distance (proportional to time). Graph 775 shows a time snapshot of the two ultrasound pressure waveforms, where the standing waves (highlighted by a circle) are limited to the region where the pressure envelopes overlap. Graph 780 shows the pressure envelopes when the foci of the two transducers are positioned at different distances, and graph 785 shows the corresponding interference pattern, illustrating how spatially limited standing waves are formed.

In some embodiments, the system 100 may selectively adapt the focal depths, angular poses, and ultrasound parameters to fine-tune the extent and intensity of standing waves. Preferably, the standing wave region is confined to avoid widespread modulation across multiple wavelengths, thereby enhancing the precision and efficacy of neuromodulation.

Additionally, reflections from anatomical structures, such as the skull, may also contribute to standing wave formation. To control and minimize unintended standing wave regions, the system 100 may adjust parameters such as the wavefront incidence angle and numerical aperture (NA), using larger NA values to shorten focal zones and further limit standing wave spatial extent.

Thus, when standing waves are employed as a mechanism for ultrasonic neuromodulation, methods to spatially constrain their formation are preferred to reduce off-target effects and to maximize the targeting specificity within the brain.

Antiparallel stretching

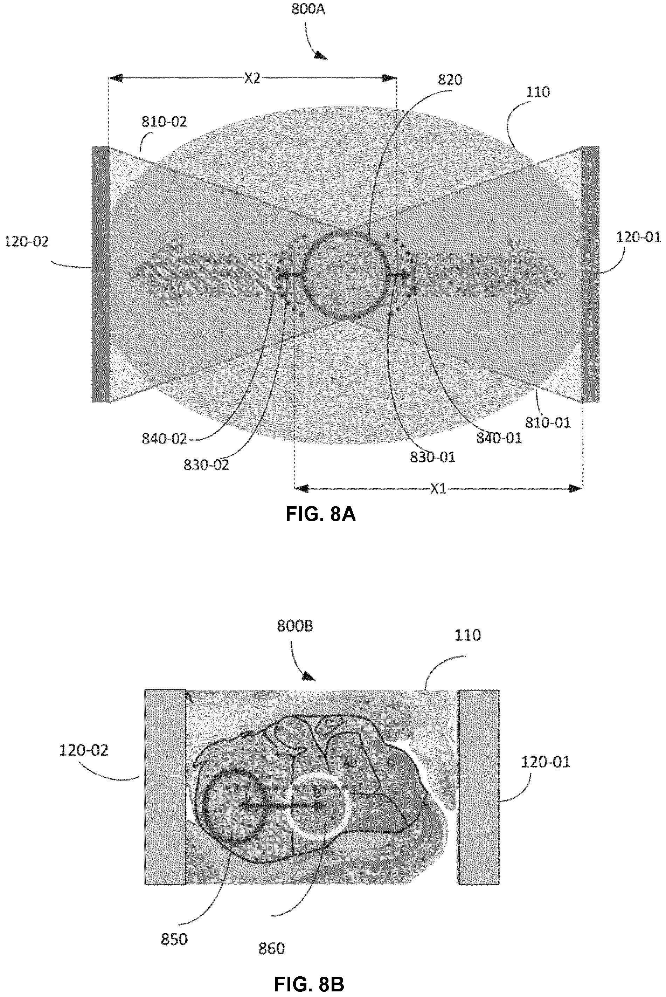

Figs. 8A and 8B illustrate examples of transcranial ultrasound stimulation fields configured to induce antiparallel stretching within a brain region or between brain regions, according to embodiments of the invention.

In contrast to approaches that utilize standing waves for neuromodulation, in some embodiments, the system 100 may be configured to generate antiparallel ultrasound fields focused beyond each other, producing opposing acoustic radiation forces that result in targeted stretching of neural structures. This stretching effect may be achieved without forming standing waves, by applying suitable angular offset between wavefronts and/or by operating the transducers at different ultrasound frequencies.

Fig. 8A shows an schematic representation 800A of the head 110 of a subject with a circular target region 820. Two ultrasound transducers 120-01 and 120-02 are positioned on opposing sides of the subject's head. Each transducer generates an ultrasound field (fields 810-01 and 810-02, respectively), with focal depths X1 and X2 set beyond the target region 820. Thus, the ultrasound fields have local depths configured to provide focal points/regions arranged beyond each other within the target brain region, thereby forming a limited overlapping space between said focal points/regions. This configuration results in acoustic radiation forces 840-01 and 840-02 directed toward one another from opposite sides, generating strain within the overlapping region/space of the target 820. The opposing acoustic fields thus induce a targeted stretching effect within the brain tissue enclosed by the overlapping region, without relying on standing wave interference.

The control unit 130 may be configured to determine the angular pose of the ultrasound transducers 120-01, 120-02 such that they are not positioned exactly in opposition, or they may be operated at different ultrasound frequencies, or both. These measures reduce or eliminate the formation of standing waves while preserving effective field interaction within the target region. The focal points/regions of the ultrasound fields define the limited overlapping space, within which the ultrasound energy intersects, enabling spatially controlled antiparallel stretching of the tissue. Such spatially confined strain may enhance mechanical modulation of neuronal activity while reducing the likelihood of off-target effects.

Fig. 8B illustrates another schematic representation 800B of an embodiment in which antiparallel ultrasound stimulation is applied to induce separation between two brain regions 850 and 860. Ultrasound transducers 120-01 and 120-02 are positioned to generate opposing ultrasound fields, which create directional strain across the tissue bridging the two regions. This stretching configuration may be advantageous for modulating locally connected brain regions, in addition to those connected via white matter tracts.

In both cases, the control unit 130 may be configured to determine the ultrasound stimulation parameters, including (inter alia) the focal depths, transducer placement, angular pose, and operating frequencies, to generate coordinated antiparallel ultrasound fields that maximize the mechanical/stimulation effects within the targeted region. Preferably, the parameters are selected such that the standing wave formation is suppressed and the spatial extent of the mechanical strain is confined to the intended neural structures.

Stretch and strain within a single brain region

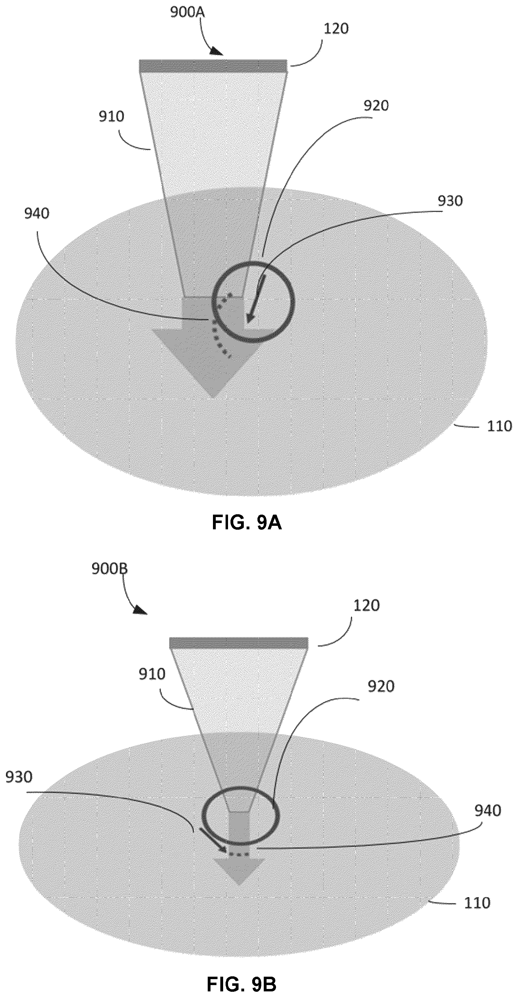

Figs. 9A to 9E illustrate various embodiments in which the transcranial ultrasound stimulation system 100 generates ultrasound fields to induce mechanical strain, such as lateral shearing or compressive displacement, within a single target brain region 920. In particular, the control unit 130 is configured to generate the corresponding ultrasound parameters enabling the system 100 to generate said ultrasound fields.

In these embodiments, one or more ultrasound transducers may be used to apply ultrasound fields to opposite or adjacent sides of the target region, either alternately or simultaneously. The stimulation parameters, including the angle of incidence and temporal patterning of the ultrasound fields, may be determined by the control unit to optimize the type and localization of the induced mechanical effects.

Fig. 9A shows a configuration 900A in which an ultrasound transducer 120 of system 100 generates a narrow ultrasound beam 910 that approaches the target brain region 920 laterally. The ultrasound field applies a shearing force from one side of the region, resulting in lateral shearing strain 940 within the tissue.

Fig. 9B illustrates a configuration 900B in which the ultrasound transducer 120 generates a narrow beam 910 directed vertically downward toward the center of a narrow target region 920. This configuration induces a compressive displacement, producing localized strain within the tissue.

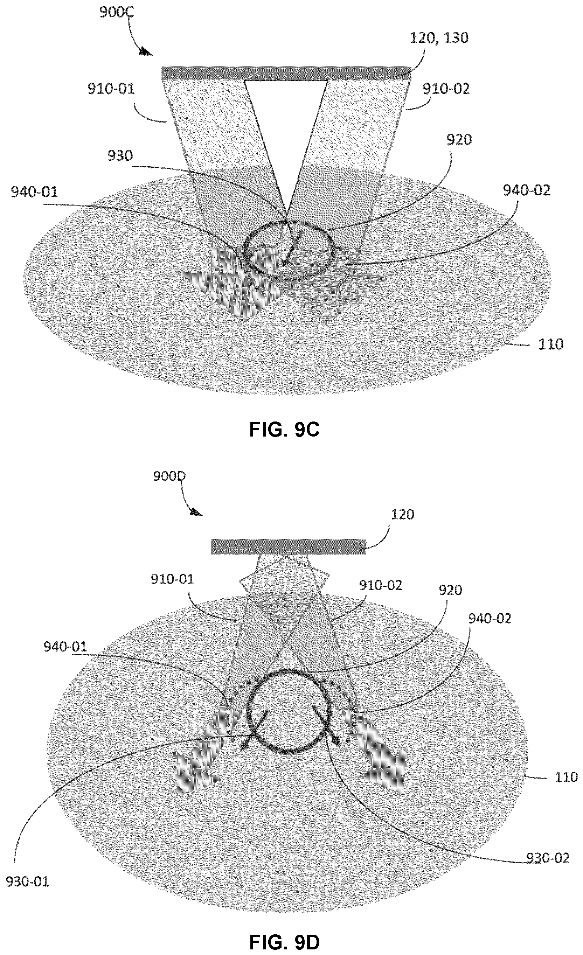

Fig. 9C shows an embodiment 900C in which two ultrasound beams are directed alternately at opposite lateral sides of the target brain region 920. The beams may be generated by two separate transducers or steered from a common source (e.g. a single ultrasound transducer 120). The alternating application of ultrasound fields results in cyclic stretching of the tissue, with strain 940-01 induced on the left side of the region and strain 940-02 induced on the right. This configuration produces a back-and-forth shearing motion within the region.

Fig. 9D illustrates a configuration 900D in which a single ultrasound transducer 120 generates steerable ultrasound beams that are alternately directed toward different lateral sides of the target brain region 920. The alternate wavefront directions are indicated as 910-01 and 910-02, targeting the left and right sides, respectively. This approach enables dynamic control of strain using a single transducer by electronically steering the acoustic field. The alternating or sweeping pattern of stimulation may generate cyclic lateral shearing within the tissue. Beam steering may be achieved using electronic phasing of an ultrasound transducer array or mechanical adjustment, wherein the control unit 130 may be configured to control any of these functions (e.g. by means of determining the ultrasound stimulation parameters, which may include function-related parameters, such as applicable steering angle, and/or steering speed or steering frequency).

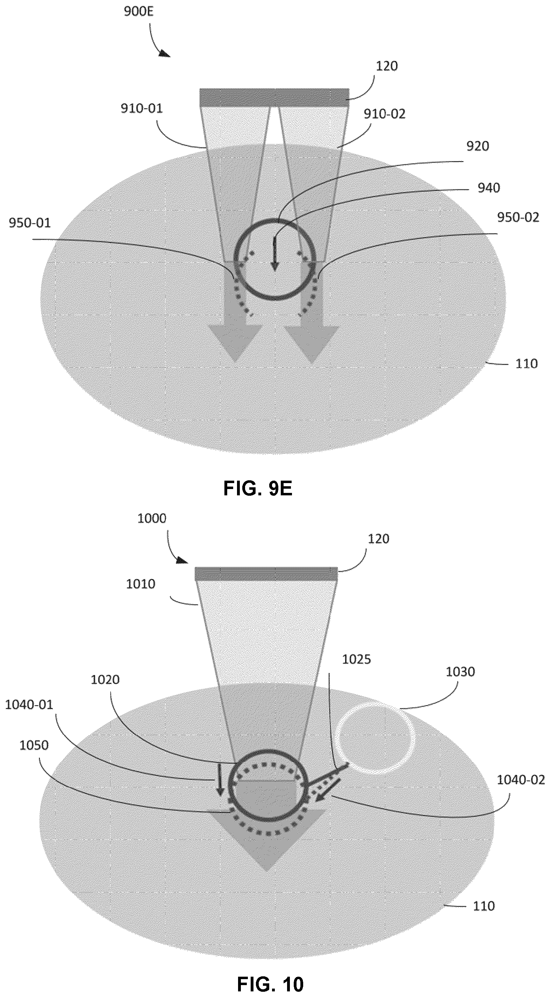

Fig. 9E shows an embodiment 900E in which simultaneous application of ultrasound fields is used to stretch the lateral edges of the brain region 920. In this configuration, the wavefronts approach the region from opposite sides at non-glancing angles, such that the mechanical effect is concentrated within the tissue volume, rather than distributed along the surface. This configuration may enhance strain localization and field penetration.

In some embodiments, the control unit 130 may be configured to control the stimulation parameters, beam geometry, and timing such that the ultrasound fields induce lateral shearing - that is, relative displacement of adjacent tissue regions in directions parallel to the main axis of field propagation- and/or steering. Lateral shearing may generate mechanical strain patterns distinct from pure compression or extension, and may improve the specificity and efficacy of the neuromodulation within the target region.

Maximizing stretching of processes from and to deeper regions

Fig. 10 illustrates an embodiment 1000 of the transcranial ultrasound stimulation system 100 configured to target and modulate deep brain regions through the mechanical stretching of neuronal processes, such as axons or white matter tracts, that connect to other brain regions.

In this example, a deep brain region 1020 is structurally connected to a more superficial region (e.g. shallower regions) 1030 via a neural tract 1025, such as a long-range axonal projection or a white matter fiber bundle. The ultrasound transducer 120 of system 100 generates an ultrasound field or beam 1010 focused on the deep region 1020. The ultrasound field induces an acoustic radiation force 1040-01, which displaces the deep region 1020 in the direction of the left arrow towards position 1050.

As the deep target region 1020 is displaced by the applied acoustic radiation force, the moving of target 1020 towards 1050 causes the tract 1025 connecting to region 1030 to stretch and thus activate neurons in the connected brain regions, which mechanically stretches toward the more superficial region 1030. This mechanical strain may influence the functional state of neurons within the connecting tract, as well as those in the connected brain regions.

In particular, the stretching of outgoing axons or efferent tracts may produce backpropagated action potentials or activate mechanosensitive channels, leading to changes in excitability in neurons located within the deep target region 1020 or the structurally connected region 1030. This configuration may thus enable indirect modulation of brain regions that are not directly targeted by the ultrasound focus, by mechanically engaging the pathways that connect them.

In some embodiments, the system 100 (e.g. the control unit 130) may determine stimulation parameters such as focal depth, ultrasound intensity, and beam direction to selectively target the neural origin or termination point of specific axonal pathways. The anatomical data describing the spatial trajectory and connectivity of these pathways may be obtained from subject-specific imaging (e.g. diffusion tensor imaging) or retrieved from generic neuroanatomical models accessible by the control unit 130 (e.g. stored as part of the application, e.g. as a database). By focusing on a structure that is mechanically coupled to another region, the system may extend the modulation effect beyond the direct field of acoustic stimulation, allowing precise engagement of distributed brain circuits.

Collinear targeting of two regions

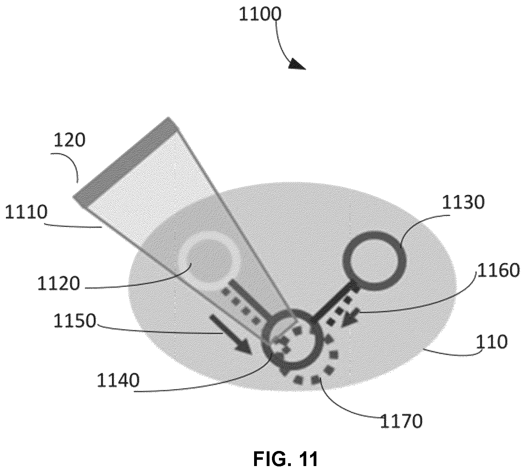

Fig. 11 illustrates an embodiment 1100 of the transcranial ultrasound stimulation system 100 configured to selectively modulate neural connections between two anatomically connected brain regions by generating a collinear mechanical force aligned with the relevant structural pathway.

In this example, brain region 1140 is connected to two other brain regions: 1120 and 1130. The system 100 is configured to modulate the neural tract or structural pathway connecting regions 1120 and 1140, while minimizing mechanical effects on the connection between regions 1140 and 1130.

To this end, an ultrasound transducer 120 generates an ultrasound field 1110 directed collinearly along the tract connecting regions 1120 and 1140. The resulting acoustic radiation force 1140 displaces region 1140 toward the position 1170, thereby inducing mechanical strain 1150 along the tract between regions 1120 and 1140. Because the direction of the applied force (illustrated by an arrow) is aligned with the anatomical trajectory of the connection between 1120 and 1140, and is not aligned with the tract connecting region 1140 to region 1130, the mechanical effect on the latter tract is minimal or absent.

In contrast, if an ultrasound transducer 120 were positioned above region 1140 to apply a perpendicular force - for example, in the configuration described in Fig. 10 - the resulting displacement of region 1140 could induce strain in both outgoing connections, thereby reducing targeting specificity.

In some embodiments, the control unit 130 may determine the stimulation parameters and beam orientation such that the acoustic radiation force is directed collinearly with a series of anatomically connected regions or pathways. These connected structures may include axonal projections, white matter tracts, fiber bundles, or spatially aligned cortical zones. The anatomical information describing these connections may be acquired from subject-specific neuroimaging data, such as magnetic resonance imaging (MRI) or diffusion tensor imaging (DTI), or may be retrieved from generic anatomical models stored in or accessible by the control unit.

By aligning the radiation force with the orientation of selected neural tracts, the system may achieve targeted modulation of specific connections while minimizing off-target strain, thereby enhancing the precision and functional specificity of mechanical neuromodulation.

Method for determining ultrasound stimulation parameters based on target anatomy

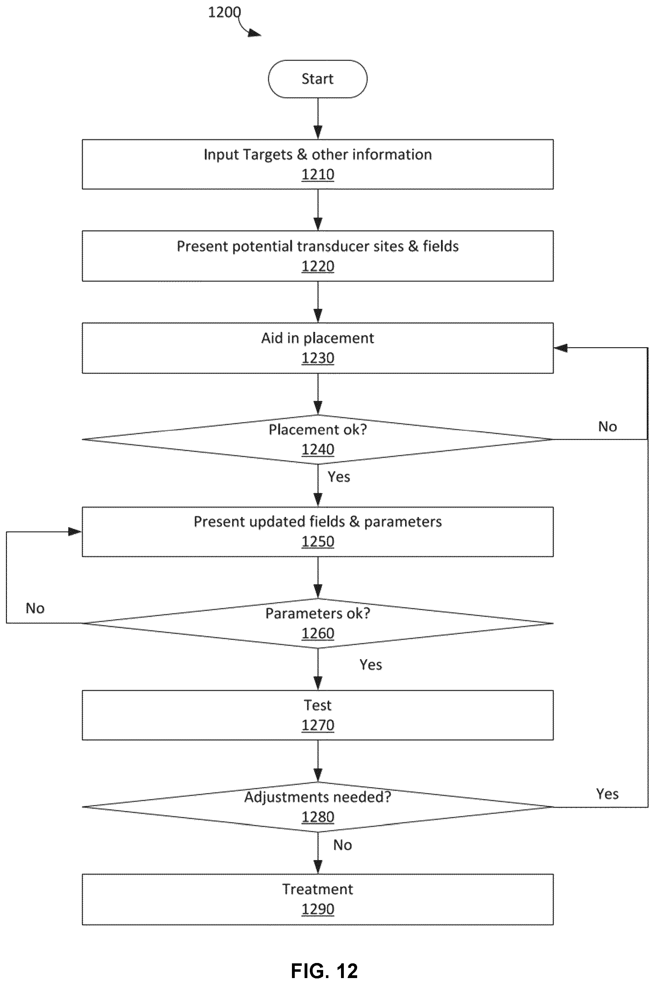

Fig. 12 illustrates a flowchart representing a method 1200 (e.g. a computer-implemented method) for configuring and executing transcranial ultrasound stimulation using the system 100, according to an embodiment of the invention. The method may be implemented by the control unit 130 in cooperation with the user interface 180, the physiological measurement system 140, and one or more ultrasound transducers 120.

In operation 1210, the system receives input (e.g. via the user interface 180) from a user, such as a clinician 190, specifying the target brain region, the stimulation objective, and optionally subject-specific information (e.g. like the subject pre-acquired anatomical imaging data -e.g. MR, CT-, and/or such as physiological data -e.g. EEG, MEG or other physiological measurement data, such as patient head measurements, or results from previous neuromodulation sessions, etc.).

In operation 1220, the system computes a set of initial ultrasound stimulation parameters based on the received input. These parameters may include one or more of: the number of ultrasound transducers 120 to be used (the number may correspond to a subset of ultrasound transducers 120 when the system 100 comprises a plurality of ultrasound transducers 120), their functional roles (e.g. stimulation and/or imaging), focal depth, pressure amplitude, stimulation timing, and field geometry. Further, the system (e.g. the control unit 130) provides proposed placement locations and angular poses for each ultrasound transducer 120. The computation/determination of ultrasound stimulation parameters may include automatic strategies to minimize off-target effects and may include gradient-based field optimization (e.g. M1, M2, or M3 metrics as previously defined). In this operation 1220, the system may also displays the ultrasound fields to Clinician 190 via the user interface 180 and presents updated target information. For example, System 100 may suggest focusing on the lateral sides of the target region and thus having two target locations for the ultrasound fields.

When the system 100 is used for two connected brain regions, especially if the distance between the regions is large, the area of stretching could be in several areas between the two regions. In this scenario, Method 1200 may suggest parameters to minimize off-target effects. In general, Method 1200 optimizes the parameters to minimize off-target effects. Method 1200 provides suggested timing parameters based on the time scale of the neuronal activity of the target brain region. Method 1200 optimizes the pressure gradient to maximize the stretching. Method 1200 could optimize the pressure gradient in one, two, or three directions. The directions correspond to the three axes: x, y, and z.

In Operation 1230, the method 1200 outputs the determined placement and angular pose for each ultrasound transducer 120 (e.g. via the user interface 180). Further, operation 1230 may further comprise providing guiding information for aiding the clinician 190 in placing the ultrasound transducers 120 on the patient's head 110 according to the determined placements and angular poses. Although the method 1200 may use a neuronavigational equipment of the system 100, a more standard operation includes providing the guiding information through the user interface 180 (e.g. via a display) to guide the clinician 190 in placing the ultrasound transducers 120. Method 1200 may also utilize physiological measurements 140 (such as EEG and/or imaging data from one or more transducers 120) for guiding purposes. Guided by the system, the clinician 190 is enabled to place the ultrasound transducers 120 on the patient's head 110.

In operation 1240, the system evaluates the current placement of the ultrasound transducers 120 based on real-time data (e.g. provided by the physiological measurement system 140) and determines whether adjustments are needed. If required, the process may loop back to operation 1230 for refinement.

Throughout the operations, in particular Operations 1220, 1230, and 1240, ultrasound field shape simulation and aberration correction algorithms may be used. The algorithms account in particular for field distortions such as lateral displacements; altering of the locations of maximal pressure, pressure gradients, or other parameters of the field; and defocusing or other smearing of the field affecting the efficacious parameters that happen due to the presence of the skull. In 1220, they may be used to suggest initial optimal placements. In 1230 and 1240, they may use real time location of the transducer as well as ultrasonic imaging and measurements to further refine the aberration corrections.

Once placement is verified, operation 1250 presents the updated ultrasound stimulation parameters and corresponding field simulation results to the clinician 190 (e.g. via the user interface 180). These may include expected field distributions, focal region location, pressure profiles, and safety indicators.

In operation 1260, the clinician may evaluate the correctness of the parameters manually or using the evaluation algorithms that may be part of the application executed by the control unit 130. The evaluation determines that the parameters are not correct, then method apply system-suggested refinements, for example, based on pre-programmed safety thresholds or anatomical targeting objectives.

In operation 1270, the system delivers one or more test pulses or waveforms to evaluate field performance, placement and physiological response (e.g. by means of a measuring with the physiological measurement system 140). This operation allows for any corrections, for example, caused by skull aberrations, reflections, or any required changes to foci, etc. Several strategies are employed for this operation. For example, a first transducer emits test waveforms, and the results are evaluated by the physiological measurement system 140 (e.g. by measuring with one or more auxiliar ultrasound transducers, or measuring using EEG and/or EMG). In a different example, the receive elements of the same first transducer are used for imaging and evaluating the results (e.g. the first transducer may be regarded as a functional part of the physiological measurement system when acting in this configuration).

In operation 1280, feedback from the test stimulation is used to refine the ultrasound stimulation parameters and/or to refine the placement and/or angular pose of the transducers. Adjustments may include phase and/or frequency tuning to match neuronal oscillations, correction of focal distortions due to skull-induced aberrations, or updates to transducer alignment. For example, based on the timing of neuronal activity of the target region (measured using EEG 140), the frequency and phase of the ultrasound waveforms need modification. System 100 may phase lock to the signal detected by EEG 140.

In operation 1290, once the final parameter set is confirmed, the system executes the transcranial ultrasound stimulation according to the configured parameters.

The steps of the method 1200 may be performed sequentially or iteratively, and certain operations (e.g. 1230-1240 or 1250-1260) may involve repeated refinements based on user input or automated evaluation loops. The method may further incorporate continuous simulations or real-time physiological feedback to maintain precision and safety throughout the setup and stimulation phases.

It should be noted that method 1200, as illustrated in Fig. 12, represents a complete method for controlling a transcranial ultrasound stimulation system, and may be implemented in full or in part depending on the system configuration and clinical workflow. In some embodiments, the method may comprise a simplified sequence including operations 1210 (input collection), 1220 (initial parameter computation), 1230 (transducer placement assistance), 1240 (placement evaluation), and 1290 (stimulation). Other operations, such as test pulse evaluation (1270), physiological feedback-based refinement (1280), or manual adjustment steps (1260), may be optionally included to enhance targeting accuracy, safety, or efficacy depending on the available system components and the specific application scenario.

**Claims**

1. A transcranial ultrasound stimulation system (100) for controlling transcranial ultrasound stimulation, comprising:

    at least one ultrasound transducer (120) configured to be placed on a subject's (105) head (110) for transmitting ultrasound signals into brain tissue;
    a physiological measurement system (140) configured to monitor physiological data from the subject (105), the physiological data preferably comprising at least one of electroencephalography (EEG), magnetoencephalography (MEG), electromyography (EMG) signals or data captured by the at least one transducer (120); and a control unit (130) comprising at least one processor and an application comprising instructions which, when executed by the at least one processor, cause the system (100) to:

receive an input indicating a selected stimulation objective and a target brain region for receiving the stimulation;

determine ultrasound stimulation parameters for the at least one ultrasound transducer (120) based on anatomical data associated with the selected target brain region and the selected stimulation objective, the ultrasound stimulation parameters preferably comprising at least one of: focal depth, pressure to be applied, and stimulation timing;

determine a placement and an angular pose of the at least one ultrasound transducer (120), and output the determined placement and angular pose to a user via a user interface (180); and

generate control signals for the at least one ultrasound transducer (120) to transmit an ultrasound waveform causing an ultrasound field adapted to apply an ultrasound radiation force within the target brain region based on the ultrasound stimulation parameters.

2. The system (100) according to claim 1, wherein:

the control unit (130) is further configured to generate guidance information for arranging the at least one ultrasound transducer (120) at the determined placement and angular pose based on the real-time physiological data; and/or

the control unit (130) is configured to determine, and/or dynamically update, the placement and the angular pose of the at least one ultrasound transducer (120) based on real-time feedback from the physiological measurement system (140) and/or from imaging data acquired by the at least one ultrasound transducer (120).

3. The system (100) according to claim 1 or 2, wherein the control unit (130) is configured to adapt the ultrasound stimulation parameters or the control signals based on real-time feedback derived from physiological data monitored by the physiological measurement system (140), preferably wherein adapting the ultrasound stimulation parameters or the control signals comprises dynamically adjusting the frequency and/or phase of the ultrasound signals based on the real-time feedback during stimulation.

4. The system (100) according to any of the preceding claims, wherein the physiological measurement system (140) is configured to monitor a frequency and/or a phase of a neuronal activity pattern in the subject (105), and wherein the control unit (130) is configured to generate control signals for transmitting an ultrasound waveform that is phase-locked or frequency-locked to the neuronal activity pattern.

5. The system (100) according to any of the preceding claims, wherein the control unit (130) is configured to generate test control signals for the at least one ultrasound transducer (120) to transmit test ultrasound waveforms, and to refine the ultrasound stimulation parameters based on the subject's (105) physiological response to the transmitted test ultrasound waveforms monitored by the physiological measurement system (140).

6. The system (100) according to any of the preceding claims, wherein the control unit (130) is configured to determine a propagation direction of the ultrasound field based on orientation of axons or white matter tracts within the target brain region, preferably such that the ultrasound radiation force is aligned with or orthogonal to the orientation of axons or white matter tracts.

7. The system (100) according to any of the preceding claims, wherein the control unit (130) is configured to determine the ultrasound stimulation parameters so as to induce strain in a cell body anchored to a dendritic tree within the target brain region.

8. The system (100) according to any of the preceding claims, wherein the control unit (130) is configured to determine the ultrasound stimulation parameters to compensate for skull-induced ultrasound field aberrations based on a simulation of anatomical constraints and/or based on real-time measurements acquired by the physiological measurement system (140).

9. The system (100) according to any of the preceding claims, wherein the control unit (130) is configured to determine the ultrasound stimulation parameters so as to optimize a spatial pressure gradient of the ultrasound field generated by the at least one ultrasound transducer (120), by controlling an apodization function applied to elements of the ultrasound transducer (120), preferably in one or more spatial directions selected from:

a lateral or medial direction (x);
an anterior or posterior direction (y); and
a superior or inferior direction (z).

10. The system (100) according to claim 9, wherein the control unit (130) is configured to optimize the apodization so as to maximize a ratio between a spatial derivative of acoustic pressure within the ultrasound field and a maximum acoustic pressure within the ultrasound field, preferably wherein the maximization is based on a ratio selected from:

- M3, defined as:
$$M3 = \left[\left(\frac{\partial P}{\partial x}\right)^2 + \left(\frac{\partial P}{\partial y}\right)^2 + (1/c)\left(\frac{\partial P}{\partial t}\right)^2\right]/P_{max}^2$$

- M2, defined as:
$$M2 = \left[\left(\frac{\partial P}{\partial x}\right)^2 + \left(\frac{\partial P}{\partial y}\right)^2\right]/P_{max}^2 \text{ ; and}$$

- M1, defined as:
$$M1 = \left[\left(\frac{\partial P}{\partial x}\right)^2\right]/P_{max}^2$$

wherein P represents the acoustic pressure within the ultrasound field, x, y, and t represent spatial coordinates, Pmax represents a maximum amplitude of the acoustic pressure within the ultrasound field, and c represents sound speed in brain tissue.

11. The system (100) according to any of the preceding claims, comprising at least two ultrasound transducers (120), wherein the control unit (130) is configured to determine ultrasound stimulation parameters for the at least two ultrasound transducers (120) so as to coordinate the ultrasound fields generated by the at least two ultrasound transducers (120) within the target brain region.

12. The system (100) according to claim 11, wherein the control unit (130) is configured to determine the ultrasound stimulation parameters, the placement, and the angular pose of each of the at least two ultrasound transducers (120), so that the at least two ultrasound transducers (120) generate ultrasound fields directed substantially antiparallel to each other across the target brain region, thereby applying opposing acoustic radiation forces to the target brain region, wherein preferably:

the ultrasound fields have focal depths positioned short within the target brain region so as to form a limited non-overlapping space between the focal points of the two ultrasound fields where the ultrasound fields intersect with each other, wherein preferably the control unit (130) is configured to selectively adjust the ultrasound stimulation parameters such that the ultrasound fields of the at least two ultrasound transducers (120) intersect with each other in the limited non-overlapping space causing spatially limited standing waves within the target brain region between the respective focal points of the two ultrasound fields; or
the ultrasound fields have focal depths configured to provide focal points arranged beyond each other within the target brain region so as to form a limited overlapping space between the focal points, wherein the ultrasound fields of the at least two ultrasound transducers (120) intersect with each other in the limited overlapping space, thereby causing antiparallel stretching of the target brain region, wherein preferably the control unit (130) causes the at least two ultrasound transducers (120) to operate at different ultrasound frequencies and/or to vary their angular pose to suppress the formation of standing waves within the target brain region.

13. The system (100) according to any of the preceding claims, wherein the control unit (130) is configured to control at least one ultrasound transducer (120) to alternately or simultaneously apply ultrasound fields directed, preferably substantially along a same main direction, to different sides of a single target brain region so as to cyclically and/or continuously induce lateral shearing within the target brain region.

14. The system (100) according to any of the preceding claims, wherein the control unit (130) is configured to control the at least one ultrasound transducer (120) to generate a collinear ultrasound radiation force directed along a series of anatomically connected regions within the target brain region so as to induce mechanical strain within one or more specific regions of the series of anatomically connected regions.

**15.** A computer-implemented method for controlling a transcranial ultrasound stimulation system (100), which is preferably a system (100) according to any of the preceding claims, the method comprising:

receiving an input indicating a selected stimulation objective and a target brain region for receiving the stimulation;

monitoring, with a physiological measurement system (140), real-time physiological data from the subject (105);

determining ultrasound stimulation parameters for at least one ultrasound transducer (120) based on anatomical data associated with the selected target brain region and the selected stimulation objective, the ultrasound stimulation parameters preferably comprising at least one of: focal depth, pressure to be applied, and stimulation timing;

determining a placement and an angular pose of the at least one ultrasound transducer (120), and outputting the determined placement and angular pose to a user via a user interface (180);

generating and providing guidance information for arranging the at least one ultrasound transducer (120) at the determined placement and angular pose based on the real-time physiological data; and

generating control signals for the at least one ultrasound transducer (120) to transmit an ultrasound field adapted to apply an ultrasound radiation force within the target brain region based on the ultrasound stimulation parameters.

100

TX

| Beamformer 171 | → | Wave Gen 172 | → | DAC/ PWM 173 | → | Output Stage 174 |

170

120

Xducer/ Probe

130

CTRL

190

RX Data

RX

180

PM

140

160

**FIG. 1A**

110

120-01

120-xx

120-xx

120-02

120-03

105

**FIG. 1B**

FIG. 2

FIG. 3A

FIG. 3B

FIG. 4A

FIG. 4B

FIG. 5

FIG. 6

FIG. 7A

FIG. 7B

FIG. 8A

FIG. 8B

FIG. 9A

FIG. 9B

900C

120, 130

910-01

910-02

930

920

940-01

940-02

110

**FIG. 9C**

900D

120

910-01

910-02

920

940-02

940-01

110

930-01

930-02

**FIG. 9D**

FIG. 9E

FIG. 10

FIG. 11

1200

```
            ( Start )
                |
                v
+-------------------------------------+
|  Input Targets & other information  |
|               1210                  |
+-------------------------------------+
                |
                v
+-------------------------------------+
|  Present potential transducer sites |
|              & fields               |
|               1220                  |
+-------------------------------------+
                |
                v
+-------------------------------------+  <------------+
|          Aid in placement           |              |
|               1230                  |              |
+-------------------------------------+              |
                |                                    |
                v                                    |
          < Placement ok? >  ---- No --------------->+
          <     1240      >
                |
               Yes
                v
+-------------------------------------+  <----+
|  Present updated fields & parameters |      |
|               1250                  |       |
+-------------------------------------+       |
                |                             |
                v                             |
          < Parameters ok? > ---- No -------->+
          <     1260      >
                |
               Yes
                v
+-------------------------------------+
|               Test                  |
|               1270                  |
+-------------------------------------+
                |
                v
        < Adjustments needed? > ---- Yes --->
        <        1280         >
                |
               No
                v
+-------------------------------------+
|            Treatment                |
|               1290                  |
+-------------------------------------+
```

**FIG. 12**

FIG. 13A

FIG. 13B

FIG. 13C

**FIG. 13D**

**FIG. 13E**

| | Europäisches Patentamt European Patent Office Office européen des brevets | **EUROPEAN SEARCH REPORT** | Application Number EP 25 17 4569 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/278199 A1 (UNIV CARNEGIE MELLON [US]; HE BIN; YU KAI) 5 January 2023 (2023-01-05) | 1,3-7, 11,14,15 | INV. A61N7/00 A61B34/00 |
| Y | * the whole document * | 9,10,12, 13 | A61B5/00 |
| | ----- | | ADD. A61B34/10 |
| X | US 2013/281890 A1 (MISHELEVICH DAVID J [US]) 24 October 2013 (2013-10-24) * paragraphs [0508] - [0511], [0527], [0551], [0563], [0574], [0616], [0690], [0694]; figures 10, 18, 20, 31, 35, 46, 66 * | 1-3,11, 14,15 | |
| | ----- | | |
| X | KEITH R MURPHY ET AL: "A Practical Guide to Transcranial Ultrasonic Stimulation from the IFCN-endorsed ITRUSST Consortium", ARXIV.ORG, CORNELL UNIVERSITY LIBRARY, 201 OLIN LIBRARY CORNELL UNIVERSITY ITHACA, NY 14853, 10 July 2024 (2024-07-10), XP091815091, | 1,5-8, 11,14,15 | |
| Y | * the whole document * | 3 | TECHNICAL FIELDS SEARCHED (IPC) |
| | ----- | | A61B A61N |
| Y | WO 2024/090876 A1 (KERI KOREA ELECTROTECHNOLOGY RES INST [KR]) 2 May 2024 (2024-05-02) * paragraphs [0051], [0075] - [0076]; figure 6c * | 9,10 | |
| | ----- | | |
| Y | US 2023/249006 A1 (KHURI-YAKUB BUTRUS T [US] ET AL) 10 August 2023 (2023-08-10) * paragraphs [0041], [0105]; figures 1A-1C, 10B * | 12 | |
| | ----- | | |
| Y | US 2022/379142 A1 (EISAMAN MATTHEW DIXON [US] ET AL) 1 December 2022 (2022-12-01) | 3,13 | |
| A | * the whole document * | 1,4-12, 14 | |
| | ----- | | |

The present search report has been drawn up for all claims

| Place of search | Date of completion of the search | Examiner |
|---|---|---|
| The Hague | 9 October 2025 | Friedrich, Franz |

EPO FORM 1503 03.82 (P04C01)

## ANNEX TO THE EUROPEAN SEARCH REPORT
## ON EUROPEAN PATENT APPLICATION NO.

EP 25 17 4569

This annex lists the patent family members relating to the patent documents cited in the above-mentioned European search report.
The members are as contained in the European Patent Office EDP file on
The European Patent Office is in no way liable for these particulars which are merely given for the purpose of information.

09-10-2025

| Patent document cited in search report | Publication date | Patent family member(s) | Publication date |
|---|---|---|---|
| WO 2023278199 A1 | 05-01-2023 | US 2025073502 A1<br>WO 2023278199 A1 | 06-03-2025<br>05-01-2023 |
| US 2013281890 A1 | 24-10-2013 | NONE | |
| WO 2024090876 A1 | 02-05-2024 | KR 20240059464 A<br>WO 2024090876 A1 | 07-05-2024<br>02-05-2024 |
| US 2023249006 A1 | 10-08-2023 | NONE | |
| US 2022379142 A1 | 01-12-2022 | NONE | |

EPO FORM P0459

For more details about this annex : see Official Journal of the European Patent Office, No. 12/82